# EUROPÄISCHE PATENTSCHRIFT

(11) **EP 1 699 427 B1**
(45) Veröffentlichungstag und Bekanntmachung des Hinweises auf die Patenterteilung: **27.02.2008**
(21) Anmeldenummer: 04797906.7
(22) Anmeldetag: 15.11.2004
(51) Int. Cl.: A61K 8/49, A61K 8/41, A61Q 5/10

(54) **MITTEL UND VERFAHREN ZUM OXIDATIVEN FÄRBEN VON KERATINFASERN**
AGENT AND METHOD FOR OXIDATIVELY DYING KERATIN FIBERS
AGENT ET PROCEDE DE COLORATION OXYDATIVE DE FIBRES KERATINIQUES

(30) Priorität: 16.12.2003 DE 10358878
(43) Veröffentlichungstag der Anmeldung: 13.09.2006
(73) Patentinhaber: Wella Aktiengesellschaft, 64274 Darmstadt (DE)
(72) Erfinder: PASQUIER, Cécile, CH-1723 Marly (CH); BUCLIN, Véronique, CH-1638 Morlon (CH); KIENER, Caroline, CH-1731 Ependes (CH); DUC-REICHLIN, Nadja, CH-1470 Lully (CH); BRAUN, Hans-Jürgen, CH-3182 Ueberstorf (CH)
(86) Internationale Anmeldenummer: PCT/EP2004/012942
(87) Internationale Veröffentlichungsnummer: WO 2005/060928

(56) Entgegenhaltungen:
- WO-A-02/074268
- AT-B- 282 072
- DE-A1- 1 922 400
- DE-A1- 19 856 342

## Beschreibung

Gegenstand der vorliegenden Anmeldung ist ein Mittel zur Färbung von Keratinfasern, wie zum Beispiel Seide, Wolle oder Haaren und insbesondere menschlichen Haaren, welches (i) ein heterozyklisches Hydrazon-Derivat, (ii) ein aromatisches Enamin und (iii) ein Oxidationsmittel enthält, ein Mehrkomponenten-Kit sowie ein Verfahren zum Färben von Keratinfasern unter Verwendung dieses Färbemittels.

Haarfärbemittel werden je nach zu färbender Ausgangshaarfarbe und gewünschtem Endresultat hauptsächlich in die Gruppe der Oxidationsfärbemittel oder der Tönungen unterteilt. Oxidationsfärbemittel eignen sich hervorragend für die Abdeckung von höheren Grauanteilen, hierbei werden die bei einem Grauanteil von bis zu 50 % verwendeten Oxidationsfärbemittel in der Regel als oxidative Tönungen bezeichnet, während die bei einem Grauanteil von über 50 % oder zum "Hellerfärben" verwendeten Oxidationsfärbemittel in der Regel als sogenannte oxidative Farben bezeichnet werden. Direktziehende Farbstoffe sind hauptsächlich in nicht-oxidativen Färbemitteln (sogenannten Tönungsmitteln) enthalten. Einige direktziehende Farbstoffe, wie zum Beispiel Nitrofarbstoffe, können aufgrund ihrer geringen Größe in das Haar eindringen und es -zumindest in den äusseren Bereichen- direkt anfärben. Derartige Tönungen sind sehr haarschonend und überstehen in der Regel 6 bis 8 Haarwäschen. Direktziehende Farbstoffe werden ebenfalls oft in oxidativen Färbemitteln zur Erzeugung bestimmter Nuancen beziehungsweise zur Intensivierung der Farbe eingesetzt.

Aus der DE-A 1 922 400 ist die Verwendung von Hydrazonen zur Färbung von Keratinfasern bekannt. Diese Färbemittel können jedoch die an Färbemittel gestellten Anforderungen nicht in jeder Hinsicht, insbesondere im Hinblick auf Glanz und Intensität der Färbungen, erfüllen. Überraschenderweise wurde nunmehr gefunden, dass bei Verwendung einer Kombination aus bestimmten heterozyklischen Hydrazonen und bestimmten aromatischen Enaminen in Gegenwart eines Oxidationsmittels intensive und brillante Färbungen erhalten werden.

Die internationale Patentanmeldung WO 02/074268 offenbart Oxidationsfärbemittel für Keratinfasern, die als Entwickler ein bestimmtes Hydrazon-Derivat und als Kuppler heterozyklische Verbindungen (z.B. Indole) enthalten.

Die deutsche Offenlegungsschrift DE 198 56 342 A1 offenbart die Verwendung von durch Reaktion aromatischer Enamine (z.B. Fischer Basen) mit Aldehyden erhaltenden kationischen Polymethinfarbstoffen zur schonenden Färbung von Keratinfasern ohne den Einsatz von Oxidationsmitteln.

Gegenstand der vorliegenden Erfindung ist daher ein gebrauchsfertiges Mittel zur Färbung von Keratinfasern, wie zum Beispiel Wolle, Seide oder Haaren und insbesondere menschlichen Haaren, welches dadurch gekennzeichnet ist, dass es (a) mindestens ein Hydrazon-Derivat der Formel (I) oder dessen physiologisch verträgliches Salz, 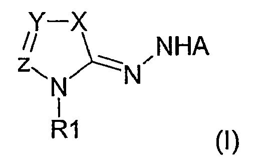 worin
**X** gleich Sauerstoff, Schwefel oder N-R2 ist,
**Y** gleich C-R3 oder Stickstoff ist und
**Z** gleich C-R4 oder Stickstoff ist,
mit der Bedingung, dass der hetererozyklische Teil der Verbindung der Formel (1) maximal drei Heteroatome enthält;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** und **R2** gleich oder verschieden sein können, und unabhängig voneinander eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, eine -C(O)-(C₁-C₁₂)-Alkylgruppe, eine substituierte oder unsubstituierte -C(O)-Phenylgruppe, eine -C(O)NH-(C₁-C₁₂)-Alkylgruppe, eine substituierte oder unsubstituierte -C(O)NH-Phenylgruppe, eine substituierte oder unsubstituierte Phenylgruppe oder eine Benzylgruppe darstellen;
**R3** und **R4** gleich oder verschieden sein können und unabhängig voneinander Wasserstoff, ein Halogenatom (F, Cl, Br, J), eine gesättigte oder ungesättigte (C₁-C₁₂)-Alkylgruppe, eine mit einem Halogenatom (F, Cl, Br, J) substituierte (C₁-C₁₂)-Alkylgruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkyl-aminogruppe, eine Di(C₁-C₁₂)-alkylaminogruppe, eine Carboxylgruppe, eine -C(O)O-(C₁-C₁₂)-Alkylgruppe, eine substituierte oder unsubstituierte -C(O)O-Phenylgruppe, eine substituierte oder unsubstituierte Phenylgruppe oder eine Naphtylgruppe darstellen;
und wenn **Y** und **Z** gleich C-R3 und C-R4 sind, **R3** und **R4** gemeinsam mit dem Restmolekül ein heterozyklisches oder carbozyklisches, gesättigtes oder ungesättigtes, substituiertes oder unsubstituiertes Ringsystem bilden können;
und (b) mindestens ein aromatisches Enamin der Formel (IIa) oder dessen Säureadditionssalze der Formel (IIb) 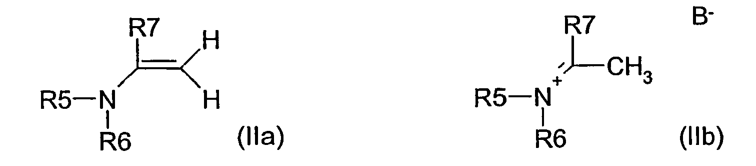
in der **R5** gleich einem ein- oder mehrkernigen aromatischen Rest, insbesondere einem gegebenenfalls mit einer (C₁-C₁₂)-Alkylgruppe, einer Monohydroxy-(C₁-C₁₂)-alkylgruppe, einer Hydroxygruppe, einer (C₁-C₁₂)-Alkoxygruppe, einer Di-(C₁-C₁₂)-alkylaminogruppe oder einer Halogengruppe substituierten 5- gliedrigen oder 6-gliedrigen Arylrest (vorzugsweise einem Phenylrest oder einem Naphthylrest), oder einem 5-gliedrigen oder 6-gliedrigen Heterozyklus (vorzugsweise einem Pyridylrest) ist;
**R6** gleich einer (C₁-C₁₂)-Alkylgruppe, einer Monohydroxy-(C₁-C₁₂)-alkylgruppe oder Mono-(C₁-C₆)-alkoxy-(C₁-C₆)-alkylgruppe, wobei zwischen den C-Atomen der Alkylkette Sauerstoffatome sitzen können, ist und
**R7** gleich einer (C₁-C₁₂)-Alkylgruppe, einer Mono-(C₁-C₆)-alkoxy-(C₁-C₆)-alkylgruppe, einer (C₁-C₆)-Alkylen-(C₁-C₆)-gruppe, einer (C₁-C₆)-Alkoxy-(C₁-C₆)-alkylengruppe, oder -O-, -NR8- oder -S- ist,
mit **R8** gleich einer (C₁-C₁₂)-Alkylgruppe, einer Mono-(C₁-C₆)-alkoxy-(C₁-C₆)-alkylgruppe, einer Monohydroxy-(C₁-C₁₂)-alkylgruppe oder Wasserstoff, wobei die Reste **R5** und **R7** gemeinsam mit dem Stickstoffatom und dem Kohlenstoffatom der Enamingrundstruktur eine zyklische Verbindung bilden können, und
**B⁻** gleich einem Anion einer organischen oder anorganischen Säure ist; und (c) mindestens ein Oxidationsmittel enthält.

Je nach dem pH-Wert des Mittels kann die Verbindung der Formel (I) auch im Gleichgewicht mit der Verbindung der Formel (la) vorliegen:

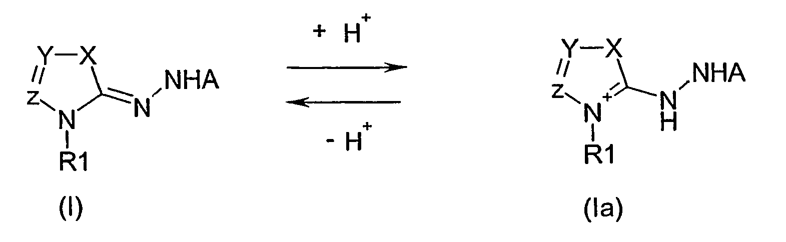

Bevorzugte Hydrazone sind Hydrazonderivate der Formel (I) oder deren physiologisch verträgliche Salze, bei denen gilt:
(i) **X** ist gleich Schwefel, **Y** ist gleich C-R3, **Z** ist gleich C-R4 und **A** stellt ein Wasserstoffatom dar, oder
(ii) **X** ist gleich N-R2, Y ist gleich Stickstoff und **A** stellt ein Wasserstoffatom;
wobei Hydrazonderivate der Formel (I) oder deren physiologisch verträgliche Salze mit **X** gleich Schwefel, Y gleich C-R3, **Z** gleich C-R4 und **A** gleich Wasserstoff besonders bevorzugt sind.

Als Beispiel für die Verbindungen der Formel (I) können die folgenden Verbindungen, sowie deren Salze, gennant werden:
3-Methyl-2(3H)-thiazolon-hydrazon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazolon-hydrazon,
4-(4-Ethoxy)phenyl-3-methyl-2(3H)-thiazolon-hydrazon,
4-(4-Bromphenyl)-3-methyl-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
3-Methyl-4-(2-naphthalenyl)-2(3H)-thiazolon-hydrazon,
2-Hydrazono-2,3-dihydro 3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
4,5-Diphenyl-3-methyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyl)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazolcarbonsäureethylester,
4-Amino-2-hydrazono-2,3-dihydro-3-methyl-5-thiazolcarbonitril,
4,5-Dimethyl-3-ethyl-2(3H)-thiazolon-hydrazon,
2-Hydrazono-2,3-dihydro-3-ethyl-4-methyl-thiazolcarbonsäureethylester,
5-Methyl-3-(1-methylethyl)-4-phenyl-2(3H)-thiazolon-hydrazon,
3-(1-Methylethyl)-4,5-diphenyl-2(3H)-thiazolon-hydrazon,
4,5-Diphenyl-3-propyl-2(3H)-thiazolon-hydrazon,
3-Butyl-4,5-diphenyl-2(3H)-thiazolon-hydrazon,
4,5-Diphenyl-3-(2-methylpropyl)- 2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Aminoethyl-2(3H)-thiazolon-hydrazon,
3-Aminoethyl-4-methyl-2(3H)-thiazolon-hydrazon,
3,4-Diphenyl-2(3H)-thiazolon-hydrazon,
4-Methyl-3-phenyl-2(3H)-thiazolon-hydrazon,
4-p-Biphenylyl-3-phenyl-2(3H)-thiazolon-hydrazon,
4-(4-Methoxy)phenyl-3-phenyl-2(3H)-thiazolon-hydrazon,
4-tert-Butyl-3-phenyl-2(3H)-thiazolon-hydrazon,
3,4-Diphenyl-5-methyl-2(3H)-thiazolon-hydrazon,
3,4,5-Triphenyl-2(3H)-thiazolon-hydrazon,
4,5-Dimethyl-3-(phenylmethyl)-2(3H)-thiazolon-hydrazon,
3-(2-Propenyl)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Diphenyl-3-(2-propenyl)-2(3H)-thiazolon-hydrazon,
2-Hydrazono-2,3-dihydro-3-[(phenylamino)carbonyl]-4-methyl-thiazolcarbonsäureethylester,
3-Methyl-4,5,6,7-tetrahydro-2(3H)-benzothiazolon-hydrazon,
3-Methyl-2(3H)-benzothiazolon-hydrazon,
3,6-Dimethyl-2(3H)-benzothiazolon-hydrazon,
6-Chlor-3-methyl-2(3H)-benzothiazolon-hydrazon,
7-Chlor-3-methyl-2(3H)-benzothiazolon-hydrazon,
6-Hydroxy-3-methyl-2(3H)-benzothiazolon-hydrazon,
5-Methoxy-3-methyl-2(3H)-benzothiazolon-hydrazon,
7-Methoxy-3-methyl-2(3H)-benzothiazolon-hydrazon,
5,6-Dimethoxy-3-methyl-2(3H)-benzothiazolon-hydrazon,
5-Ethoxy-3-methyl-2(3H)-benzothiazolon-hydrazon,
6-Ethoxy-3-methyl-2(3H)-benzothiazolon-hydrazon,
3-Methyl-5-nitro-2(3H)-benzothiazolon-hydrazon,
3-Methyl-6-nitro-2(3H)-benzothiazolon-hydrazon,
5-Acetamido-3-methyl-2(3H)-benzothiazolon-hydrazon,
6-Acetamido-3-methyl-2(3H)-benzothiazolon-hydrazon,
5-Anilino-3-methyl-2(3H)-benzothiazolon-hydrazon,
6-Anilino-3-methyl-2(3H)-benzothiazolon-hydrazon,
2-Hydrazono-2,3-dihydro-3-methyl-6-benzothiazol-carbonsäure,
2-Hydrazono-2,3-dihydro-3-methyl-4-benzothiazol-sulfonsäure,
2-Hydrazono-2,3-dihydro-3-methyl-5-benzothiazol-sulfonsäure,
2-Hydrazono-2,3-dihydro-3-methyl-6-benzothiazol-sulfonsäure,
2-Hydrazono-2,3-dihydro-3-methyl-7-benzothiazol-sulfonsäure,
2-Hydrazono-2,3-dihydro-N, N,3-trimethyl-6-benzothiazol-sulfonsäureamid,
[(2-Hydrazono-2,3-dihydro-3-methyl-6-benzothiazolyl)oxy]-essigsäure-hydrazid,
3-Methyl-naphtho[2,3-d]thiazol-2(3H)-on-hydrazon,
3-Ethyl-2(3H)-benzothiazolon-hydrazon,
6-Ethoxy-3-ethyl-2(3H)-benzothiazolon-hydrazon,
3-Propyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon,
3-p-Methylbenzyl-2(3H)-benzothiazolon-hydrazon,
2-Hydrazono-2,3-dihydro-3-(2-hydroxyethyl)-6-benzothiazol-carbonsäure,
2-Hydrazono-2,3-dihydro-6-methoxy-3(2H)-benzothiazol-propansulfonsäure,
6-Hexadecyloxy-2-hydrazono-3(2H)-benzothiazol-propansulfonsäure,
2-Oxo-3-benzothiazolin-essigsäureethylester-hydrazon,
3-Acetyl-2(3H)-benzothiazolon-hydrazon,
2-Hydrazono-3(2H)-benzothiazol-carboxaldehyd,
3-Methyl-2(3H)-oxazolon-hydrazon,
3-Phenyl-2(3H)-oxazolon-hydrazon,
3-Methyl-2(3H)-benzoxazolon-hydrazon
3-Phenyl-2(3H)-benzoxazolon-hydrazon,
1,3-Dimethyl-4-imidazolin-2-on-hydrazon,
1,3-Diethyl-4-imidazolin-2-on-hydrazon,
1,3-Dihydroxyethyl-4-imidazolin-2-on-hydrazon,
1,3-Diaminoethyl-4-imidazolin-2-on-hydrazon,
1,3-Dimethyl-4-methoxy-4-imidazolin-2-on-hydrazon,
1,3,4-Trimethyl-4-imidazofin-2-on-hydrazon,
1,3-Dimethyl-4-phenyl-4-imidazolin-2-on-hydrazon,
4-Carboxy-1,3-dimethyl-4-imidazolin-2-on-hydrazon,
4-Amino-1,3-dimethyl-4-imidazolin-2-on-hydrazon,
1,3-Dimethyl-4-dimethylamino-4-imidazolin-2-on-hydrazon,
1,3-Dimethyl-2-benzimidazolinon-hydrazon,
1,3-Diethyl-2-benzimidazolinon-hydrazon,
1,3-Dihydroxyethyl-2-benzimidazolinon-hydrazon,
1,3-Diaminoethyl-2-benzimidazolinon-hydrazon,
1,3,5-Trimethyl-2-benzimidazolinon-hydrazon,
5-Methoxy-1,3-dimethyl-2-benzimidazolinon-hydrazon,
5-Brom-1,3-dimethyl-2-benzimidazolinon-hydrazon,
4,6-Dibrom-1,3-dimethyl-2-benzimidazolinon-hydrazon,
5-Chlor-1,3-dimethyl-2-benzimidazolinon-hydrazon,
1,3-Dimethyl-5-nitro-2-benzimidazolinon-hydrazon,
1,3-Dimethyl-6-nitro-2-benzimidazolinon-hydrazon,
1,4-Dimethyl-Δ2-1,2,4-triazolin-5-on-hydrazon,
1,4-Dihydroxyethyl-Δ2-1,2,4-triazolin-5-on-hydrazon,
1,4-Diaminoethyl-Δ2-1,2,4-triazolin-5-on-hydrazon,
1,3,4-Trimethyl-Δ2-1,2,4-triazolin-5-on-hydrazon,
1,4-Dimethyl-3-phenyl-Δ2-1,2,4-triazolin-5-on-hydrazon,
1,4-Dimethyl-3-methoxy-Δ2-1,2,4-triazolin-5-on-hydrazon,
1,4-Dimethyl-3-dimethylamino-Δ2-1,2,4-triazolin-5-on-hydrazon,
4-Carboxy-1,4-dimethyl-Δ2-1,2,4-triazolin-5-on-hydrazon,
4-Amino-1,4-dimethyl-Δ2-1,2,4-triazolin-5-on-hydrazon,
4-Butyl-1-methyl-3-phenyl-Δ2-1,3,4-triazolin-5-on-hydrazon,
4-Methyl-Δ2-1,3,4-thiadiazolin-5-on-hydrazon,
4-Hydroxyethyl-Δ2-1,3,4-thiadiazolin-5-on-hydrazon,
4-Aminoethyl-Δ2-1,3,4-thiadiazolin-5-on-hydrazon,
4-Methyl-2-phenyl-Δ2-1,3,4-thiadiazolin-5-on-hydrazon,
2-Methoxy-4-methyl-Δ2-1,3,4-thiadiazolin-5-on-hydrazon,
2-Anilino-4-methyl-Δ2-1,3,4-thiadiazolin-5-on-hydrazon,
2-Amino-4-methyl-Δ2-1,3,4-thiadiazolin-5-on-hydrazon,
2-Dimethylamino-4-methyl-Δ2-1,3,4-thiadiazolin-5-on-hydrazon,
4-Methyl-2-(methylthio)-Δ2-1,3,4-thiadiazolin-5-on-hydrazon,
4-(5-Hydrazorio-4,5-dihydro-4-methyl-1,3,4-thiadiazol-2-yl)-benzensulfonyl fluorid,
4-Methyl-Δ2-1,2,4-thiadiazolin-5-on-hydrazon,
4-Hydroxyethyl-Δ2-1,2,4-thiadiazolin-5-on-hydrazon,
4-Aminoethyl-Δ2-1,2,4-thiadiazolin-5-on-hydrazon,
4-Methyl-3-phenyl-Δ2-1,2,4-thiadiazolin-5-on-hydrazon,
3-Methoxy-4-methyl-Δ2-1,2,4-thiadiazolin-5-on-hydrazon,
3-Amino-4-methyl-Δ2-1,2,4-thiadiazolin-5-on-hydrazon,
3-Dimethylamino-4-methyl-Δ2-1,2,4-thiadiazolin-5-on-hydrazon,
3-Carboxy-4-methyl-Δ2-1,2,4-thiadiazolin-5-on-hydrazon,
1,4-Dimethyl-Δ2-1,2,4-triazolin-5-on-hydrazon,
1,4-Dihydroxyethyl-Δ2-1,2,4-triazolin-5-on-hydrazon,
1,4-Aminoethyl-Δ2-1,2,4-triazolin-5-on-hydrazon,
1,3,4-Trimethyl-Δ2-1,2,4-triazolin-5-on-hydrazon,
1,4-Dimethyl-3-phenyl-Δ2-1,2,4-triazolin-5-on-hydrazon und
4-Methyl-3-phenyl-Δ2-1,2,4-triazolin-5-on-hydrazon.

Unter den Verbindungen der Formel (I) sind die folgenden Thiazolon-hydrazon-Derivate sowie deren Salze besonders bevorzugt:
3-Methyl-2(3H)-thiazolon-hydrazon,
3,4-Dimethyl-2(3H)-thiazolon-hydrazon,
4-tert-Butyl-3-methyl-2(3H)-thiazolon-hydrazon,
3-Methyl-4-phenyl-2(3H)-thiazolon-hydrazon,
3-Methyl-4-(4-tolyl)-2(3H)-thiazolon-hydrazon,
4-(4-Methoxy)phenyl-3-methyl-2(3H)-thiazolon-hydrazon,
4-(4-Ethoxy)phenyl-3-methyl-2(3H)-thiazolon-hydrazon,
4-(4-Bromphenyl)-3-methyl-2(3H)-thiazolon-hydrazon,
4-(3-Bromphenyl)-3-methyl-2(3H)-thiazolon-hydrazon,
4-(4-Chlorphenyl)-3-methyl-2(3H)-thiazolon-hydrazon,
4-(3-Chlorphenyl)-3-methyl-2(3H)-thiazolon-hydrazon,
3-Methyl-4-(4-nitrophenyl)-2(3H)-thiazolon-hydrazon,
3-Methyl-4-(3-nitrophenyl)-2(3H)-thiazolon-hydrazon,
4-([1,1'-Biphenyl]-4-yl)-3-methyl-2(3H)-thiazolon-hydrazon,
2-Hydrazono-2,3-dihydro-3-methyl-4-thiazolcarbonsäureethylester,
3,4,5-Trimethyl-2(3H)-thiazolon-hydrazon,
3,4-Dimethyl-5-phenyl-2(3H)-thiazolon-hydrazon,
3,5-Dimethyl-4-phenyl-2(3H)-thiazolon-hydrazon,
4,5-Diphenyl-3-methyl-2(3H)-thiazolon-hydrazon,
5-Ethyl-3-methyl-4-phenyl-2(3H)-thiazolon-hydrazon,
4-(4-Bromphenyl)-3-methyl-5-phenyl-2(3H)-thiazolon-hydrazon,
3-Methyl-5-phenyl-4-(4-tolyl)-2(3H)-thiazolon-hydrazon,
5-(4-Chlorphenyl)-4-phenyl-3-methyl-2(3H)-thiazolon-hydrazon,
5-(4-Chlorphenyl)-4-(4-methoxyphenyl)-3-methyl-2(3H)-thiazolon-hydrazon,
2-Hydrazono-2,3-dihydro-3,4-dimethyl-4-thiazolcarbonsäureethylester,
4-Amino-2-hydrazino-2,3-dihydro-3-methyl-5-thiazolcarbonitril,
4,5-Dimethyl-3-ethyl-2(3H)-thiazolon-hydrazon,
2-Hydrazono-2,3-dihydro-3-ethyl-4-methyl-thiazolcarbonsäureethylester,
5-Methyl-3-(1-methylethyl)-4-phenyl-2(3H)-thiazolon-hydrazon,
4,5-Diphenyl-3-(1-methylethyl)- 2(3H)-thiazolon-hydrazon,
4,5-Diphenyl-3-propyl-2(3H)-thiazolon-hydrazon,
3-Butyl-4,5-diphenyl-2(3H)-thiazolon-hydrazon,
4,5-Diphenyl-3-(2-methylpropyl)- 2(3H)-thiazolon-hydrazon,
3-(2-Propenyl)-2(3H)-thiazolon-hydrazon,
4-Methyl-3-(2-propenyl)-2(3H)-thiazolon-hydrazon,
4-tert-Butyl-3-(2-propenyl)-2(3H)-thiazolon-hydrazon,
4-Phenyl-3-(2-propenyl)-2(3H)-thiazolon-hydrazon,
4,5-Diphenyl-3-(2-propenyl)-2(3H)-thiazolon-hydrazon,
3-Hydroxyethyl-2(3H)-thiazolon-hydrazon,
3-Hydroxyethyl-4-methyl-2(3H)-thiazolon-hydrazon,
3-Aminoethyl-2(3H)-thiazolon-hydrazon,
3-Aminoethyl-4-methyl-2(3H)-thiazolon-hydrazon,
3-Phenyl-2(3H)-thiazolon-hydrazon,
4-Methyl-3-phenyl-2(3H)-thiazolon-hydrazon,
3,4-Diphenyl-2(3H)-thiazolon-hydrazon,
4-p-Biphenylyl-3-phenyl-2(3H)-thiazolon-hydrazon,
4-(4-Methoxy)phenyl-3-phenyl-2(3H)-thiazolon-hydrazon,
4-tert-Butyl-3-phenyl-2(3H)-thiazolon-hydrazon
3,4-Diphenyl-5-methyl-2(3H)-thiazolon-hydrazon,
3,4,5-Triphenyl-2(3H)-thiazolon-hydrazon,
4,5-Dimethyl-3-(phenylmethyl)-2(3H)-thiazolon-hydrazon,
2-Hydrazono-2,3-dihydro-3-[(phenylamino)carbonyl]-4-methyl-thiazolcarbonsäureethylester,
3-Methyl-4,5,6,7-tetrahydro-2(3H)-benzothiazolon-hydrazon,
3-Methyl-2(3H)-benzothiazolon-hydrazon,
3,6-Dimethyl-2(3H)-benzothiazolon-hydrazon,
6-Chlor-3-methyl-2(3H)-benzothiazolon-hydrazon,
7-Chlor-3-methyl-2(3H)-benzothiazolon-hydrazon,
6-Hydroxy-3-methyl-2(3H)-benzothiazolon-hydrazon,
5-Methoxy-3-methyl-2(3H)-benzothiazolon-hydrazon,
7-Methoxy-3-methyl-2(3H)-benzothiazolon-hydrazon,
5,6-Dimethoxy-3-methyl-2(3H)-benzothiazolon-hydrazon,
5-Ethoxy-3-methyl-2(3H)-benzothiazolon-hydrazon,
6-Ethoxy-3-methyl-2(3H)-benzothiazolon-hydrazon,
3-Methyl-5-nitro-2(3H)-benzothiazolon-hydrazon,
3-Methyl-6-nitro-2(3H)-benzothiazolon-hydrazon,
5-Acetamido-3-methyl-2(3H)-benzothiazolon-hydrazon,
6-Acetamido-3-methyl-2(3H)-benzothiazolon-hydrazon,
5-Anilino-3-methyl-2(3H)-benzothiazolon-hydrazon,
6-Anilino-3-methyl-2(3H)-benzothiazolon-hydrazon,
2-Hydrazono-2,3-dihydro-3-methyl-6-benzothiazol-carbonsäure,
2-Hydrazono-2,3-dihydro-3-methyl-4-benzothiazol-sulfonsäure,
2-Hydrazono-2,3-dihydro-3-methyl-5-benzothiazol-sulfonsäure,
2-Hydrazono-2,3-dihydro-3-methyl-6-benzothiazol-sulfonsäure,
2-Hydrazono-2,3-dihydro-3-methyl-7-benzothiazol-sulfonsäure,
2-Hydrazono-2,3-dihydro-N,N,3-trimethyl-6-benzothiazol-sulfonsäureamid,
[(2-Hydrazono-2,3-dihydro-3-methyl-6-benzothiazolyl)oxy]essigsäure-hydrazid,
3-Methyl-naphtho[2,3-d]thiazol-2(3H)-on-hydrazon,
3-Ethyl-2(3H)-benzothiazolon-hydrazon,
6-Ethoxy-3-ethyl-2(3H)-benzothiazolon-hydrazon,
3-Propyl-2(3H)-benzothiazolon-hydrazon,
3-Butyl-2(3H)-benzothiazolon-hydrazon,
3-Hexyl-2(3H)-benzothiazolon-hydrazon,
3-Hydroxyethyl-2(3H)-benzothiazolon-hydrazon,
3-Aminoethyl-2(3H)-benzothiazolon-hydrazon,
3-p-Methylbenzyl-2(3H)-benzothiazolon-hydrazon,
2,3-Dihydro-2-hydrazono-3-(2-hydroxyethyl)-6-benzothiazol-carbonsäure,
2,3-Dihydro-2-hydrazono-6-methoxy-3(2H)-benzothiazol-propansulfonsäure,
6-Hexadecyloxy-2-hydrazono-3(2H)-benzothiazol-propan-sulfonsäure,
2-Oxo-3-benzothiazolin-essigsäureethylester-hydrazon,
3-Acetyl-2(3H)-benzothiazolon-hydrazon und
2-Hydrazono-3(2H)-benzothiazol-carboxaldehyd.

Die Verbindungen der Formel (I) sind zum Teil im Handel erhältlich. Sie können jedoch auch nach aus der Literatur bekannten Syntheseverfahren, beispielsweise der Vorschrift in Research Disclosure 174, Seite 42 - 44 (1978), oder in Analogie zu den in der DE-B 1 049 381 beschriebenen Verfahren hergestellt werden.

Bevorzugte aromatische Enamine sind aromatische Enamine der Formel (IIa) oder deren physiologisch verträgliche Säureadditionssalze der Formel (llb), bei denen die Reste **R5** und **R7** gemeinsam mit dem Stickstoffatom und dem Kohlenstoffatom der Enamingrundstruktur eine zyklische Verbindung (insbesondere einen 5- oder sechsgliedrigen Zyklus) bilden, wobei vorzugsweise **R7** am aromatischen Rest **R5** mit dem Kohlenstoffatom verbunden ist, der in ortho-Stellung zum Enaminsubstituierten Kohlenstoff steht.

Als Säureadditionssalze des Formel (IIb) sind insbesondere solche zu nennen, bei denen **B**⁻ gleich Chlorid, Bromid, Jodid, Sulfat, Hydrogensulfat, Toluolsulfonat, Benzolsulfonat, Monomethylsulfat, Hexafluorophosphat, Hexafluoroantimonat, Tetrafluoroborat, Tetraphenylborat, Formiat, Acetat oder Propionat ist, wobei das Chloridion, dasTetrafluoroboration, das Acetation und das Hydrogensulfation besonders bevorzugt sind.

Als aromatische Enamin der Formel (IIa) oder deren Säureadditionssalze der Formel (IIb) können insbesondere die folgenden Verbindungen genannt werden:
1,3,3-trimethyl-2-methylen-indolin sowie dessen Salze,
1,3-Dimethyl-3-ethyl-2-methylen-indolin sowie dessen Salze,
3,3-Dimethyl-1-(2-hydroxyethyl)- 2-methylen-indolin sowie dessen Salze,
1,3,3,4-Tetramethyl-2-methylen-indolin sowie dessen Salze,
1,3,3,5-Tetramethyl-2-methylen-indolin sowie dessen Salze,
1,3,3,6-Tetramethyl-2-methylen-indolin sowie dessen Salze,
1,3,3,7-Tetramethyl-2-methylen-indolin sowie dessen Salze,
1,3,3,6,7-Pentamethyl-2-methylen-indolin sowie dessen Salze,
1,3,3,5,7-Pentamethyl-2-methylen-indolin sowie dessen Salze,
1,3,3,4,7-Pentamethyl-2-methylen-indolin sowie dessen Salze,
5-Chloro-1,3,3-trimethyl-2-methylen-indolin oder dessen Salze,
5-Fluoro-1,3,3-trimethyl-2-methylen-indolin oder dessen Salze,
5-Isopropyl-1,3,3-trimethyl-2-methylen-indolin oder dessen Salze,
5-Hydroxy-1,3,3-trimethyl-2-methylen-indolin sowie dessen Salze,
5-Methoxy-1,3,3-trimethyl-2-methylen-indolin sowie dessen Salze,
3,3-Dimethyl-1-ethyl-5-methoxy-2-methylen-indolin sowie dessen Salze,
5-Nitro-1,3,3-trimethyl-2-methylen-indolin sowie dessen Salze,
5-Amino-1,3,3-trimethyl-2-methylen-indolin sowie dessen Salze,
5-N-acetylamino-1,3,3-trimethyl-2-methylen-indolin sowie dessen Salze,
6-Hydroxy-1,3,3-trimethyl-2-methylen-indolin sowie dessen Salze,
6-Methoxy-1,3,3-trimethyl-2-methylen-indolin sowie dessen Salze
5-Methoxy-6-nitro-1,3,3-trimethyl-2-methylen-indolin sowie dessen Salze,
5-Methoxy-6-amino-1,3,3-trimethyl-2-methylen-indolin sowie dessen Salze,
5-Methoxy-6-N-acetylamino-1,3,3-trimethyl-2-methylen-indolin sowie dessen Salze,
5,6-Dihydroxy-1,3,3-trimethyl-2-methylen-indolin sowie dessen Salze,
5,6-Dimethoxy-1,3,3-trimethyl-2-methylen-indolin sowie dessen Salze,
5,6-Methylendioxy-1,3,3-trimethyl-2-methylen-indolin sowie dessen Salze,
4,5-Dihydroxy-1,3,3-trimethyl-2-methylen-indolin sowie dessen Salze,
5,7-Dihydroxy-1,3,3-trimethyl-2-methylen-indolin sowie dessen Salze,
5-Amino-6-methoxy-1,3,3-trimethyl-2-methylen-indolin sowie dessen Salze,
5-Amino-7-hydroxy-1,3,3-trimethyl-2-methylen-indolin sowie dessen Salze,
7-Amino-5-hydroxy-1,3,3-trimethyl-2-methylen-indolin sowie dessen Salze,
7-N-Acetylamino-5-hydroxy-1,3,3-trimethyl-2-methylen-indolin sowie
dessen Salze, 1,1,2,3-Tetramethyl-1H-benz[e]indolinium-Salze,
2,3-Dimethylbenzothiazolium Salze und 3-Ethyl-2-methyl-benzothiazolium Salze,
wobei die folgenden Verbindungen besonders bevorzugt sind:
1,2,3,3-Tetramethyl-3H-indolium-chlorid,
1,2,3,3-Tetramethyl-3H-indolium-bromid,
1,2,3,3-Tetramethyl-3H-indolium-sulfat,
1,2,3,3-Tetramethyl-3H-indolium-tetrafluorborat,
3-Ethyl-1,2,3-trimethyl-3H-indolium-chlorid,
3-Ethyl-1,2,3-trimethyl-3H-indolium-bromid,
3-Ethyl-1,2,3-trimethyl-3H-indolium-sulfat,
3-Ethyl-1,2,3-trimethyl-3H-indolium-tetrafluorborat,
1-Ethyl-5-methoxy-2,3,3-trimethyl-3H-indolium-chlorid,
1-Ethyl-5-methoxy-2,3,3-trimethyl-3H-indolium-bromid,
1-Ethyl-5-methoxy-2,3,3-trimethyl-3H-indolium-sulfat,
1-Ethyl-5-methoxy-2,3,3-trimethyl-3H-indolium-tetrafluorborat,
5-Methoxy-1,2,3,3-tetramethyl-3H-indolium-chlorid,
5-Methoxy-1,2,3,3-tetramethyl-3H-indolium-bromid,
5-Methoxy-1,2,3,3-tetramethyl-3H-indolium-sulfat,
5-Methoxy-1,2,3,3-tetramethyl-3H-indolium-tetrafluorborat,
5-Nitro-1,2,3,3-tetramethyl-3H-indolium-chlorid,
5-Nitro-1,2,3,3-tetramethyl-3H-indolium-bromid,
5-Nitro-1,2,3,3-tetramethyl-3H-indolium-sulfat und
5-Nitro-1,2,3,3-tetramethyl-3H-indolium-tetrafluorborat,
2,3-Dimethylbenzothiazolium chorid,
2,3-Dimethylbenzothiazolium bromid,
2,3-Dimethylbenzothiazolium iodid,
2,3-Dimethylbenzothiazolium methylsulfat,
3-Ethyl-2-methyl-benzothiazolium chlorid,
3-Ethyl-2-methyl-benzothiazolium bromid,
3-Ethyl-2-methyl-benzothiazolium iodid,
3-Ethyl-2-methyl-benzothiazolium methylsulfat und
3-Ethyl-2-methyl-benzothiazolium p-toluolsulfonat.

Das erfindungsgemäße Färbemittel wird in Verbindung mit einem Oxidationsmittel verwendet. Als Oxidationsmittel kommen die in Haarfärbemittel üblicherweise verwendeten Oxidationsmittel, wie zum Beispiel Wasserstoffperoxid oder dessen Anlagerungsprodukte, Persalze wie Persulfatsalze und Perboratsalze, oder Persäuren sowie enzymatische Oxidationssysteme, aber auch die Luftoxidation in Betracht.

Als bevorzugte Oxidationsmittel sind Wasserstoffperoxid oder dessen Anlagerungsprodukte (z.B. Natriumpercarbonat, Harnstoffperoxid etc.), und die Persalze wie Persulfatsalze und Perboratsalze, beispielsweise Kaliumpersulfat, Natriumpersulfat oder Ammoniumpersulfat sowie deren Mischungen, in Betracht.

Die Oxidationsmittel sind in dem gebrauchsfertigen Färbemittel (A) in einer Gesamtmenge von etwa 0,01 bis 10 Gewichtsprozent, vorzugsweise etwa 0,1 bis 5 Gewichtsprozent, enthalten.

Weiterhin kann das erfindungsgemäße Färbemittel zusätzlich zu den Verbindungen der Formel (I) sowie den Verbindungen der Formel (IIa) oder (IIb) zusätzlich weitere übliche, physiologisch unbedenkliche, direktziehende Farbstoffe aus der Gruppe der kationischen und anionischen Farbstoffe, der Dispersionsfarbstoffe, der Azofarbstoffe, der Chinonfarbstoffe und der Triphenylmethanfarbstoffe enthalten.

Die direktziehenden Farbstoffe sind in dem gebrauchsfertigen Färbemittel (A) in einer Menge von etwa 0,01 bis 10 Gewichtsprozent, vorzugsweise etwa 0,1 bis 5 Gewichtsprozent, enthalten.

Die Verbindungen der Formel (1) sowie die Verbindungen der Formel (IIa) oder (IIb) sind in dem gebrauchsfertigen Färbemittel (A) jeweils in einer Gesamtmenge von etwa 0,01 bis 10 Gewichtsprozent, vorzugsweise etwa 0,1 bis 5 Gewichtsprozent, enthalten.

Die Verbindungen der Formel (I) und die Verbindungen der Formel (IIa) oder (IIb) werden in der Regel getrennt voneinander aufbewahrt und erst kurz vor der Anwendung miteinander vermischt und mit dem Oxidationsmittel versetzt. Es ist jedoch auch möglich, sofern die Verbindungen der Formel (1), die Verbindungen der Formel (IIa) oder (IIb) und das Oxidationsmittel in fester Form vorliegen, diese gemeinsam abzupacken und das gebrauchsfertige Färbemittel (A) kurz vor der Anwendung durch Vermischen der Verbindungen der Formel (I), der Verbindungen der Formel (IIa) oder (IIb) und des Oxidationsmittels mit Wasser oder einer die übrigen Bestandteile des Mittels enthaltenden flüssigen Zubereitung herzustellen. Ebenfalls ist es möglich, sofern die Verbindungen der Formel (I) und die Verbindungen der Formel (IIa) oder (IIb) in fester Form vorliegen, diese gemeinsam abzupacken und das gebrauchsfertige Färbemittel (A) kurz vor der Anwendung durch Vermischen der Verbindungen der Formel (I) und der Verbindungen der Formel (IIa) oder (IIb) mit dem Oxidationsmittel herzustellen.

Das erfindungsgemäße Färbemittel besteht somit in der Regel aus mehreren Komponenten, welche vor der Anwendung miteinander vermischt werden. Vorzugsweise liegt das Mittel in Form eines 2-Komponenten-Kits, bestehend aus einer Farbträgermasse (A1), welche die Verbindungen der Formel (I) enthält, und einer weiteren Farbträgermasse (A2), welche die Verbindungen der Formel (IIa) oder (IIb) und gegebenenfalls ein Oxidationsmittel enthält, oder eines 3-Komponenten-Kits, bestehend aus einer Farbträgermasse (A1), welche die Verbindungen der Formel (I) enthält, einer weiteren Farbträgermasse (A2), welche die Verbindungen der Formel (IIa) oder (IIb) enthält, und einer ein Oxidationsmittel enthaltenden 3. Komponente (A3), vor.

Besonders bevorzugt ist ein 3-Komponenten-Kit, bestehend aus einer Farbträgermasse (A1), welche die Verbindungen der Formel (I) enthält, einer weiteren Farbträgermasse (A2), welche die Verbindungen der Formel (IIa) oder (IIb) enthält, und einer ein Oxidationsmittel enthaltenden 3. Komponente (A3).

Ein weiterer Gegenstand der vorliegenden Erfindung ist ein Mehrkomponenten-Kit, bestehend aus einem Mittel der Komponente (A1) und einem Mittel der Komponente (A2), wobei das Oxidationsmittel auch getrennt von der Komponente (A2) als Komponente (A3) abgepackt sein kann, sowie gegebenenfalls einem Mittel zur Einstellung des pH-Wertes (Alkalisierungsmittel oder Säure). Selbstverständlich können auch die Mittel der Komponenten (A1) und (A2) aus mehreren Einzelkomponenten bestehen, welche erst unmittelbar vor der Anwendung miteinander vermischt werden.

Ebenfalls ist ein 2-Komponenten-Kit möglich, dessen 1. Komponente aus einem die Verbindungen der Formel (I), die Verbindungen der Formel (IIa) oder (IIb) und gegebenenfalls ein Oxidationsmittel, sofern die Verbindungen der Formel (I), die Verbindungen der Formel (IIa) oder (IIb) und das Oxidationsmittel in fester Form vorliegen, sowie gegebenenfalls weitere übliche pulverförmige kosmetische Zusatzstoffe enthaltenden Pulver besteht, und dessen 2. Komponente Wasser oder eine flüssige kosmetische Zubereitung ist. Bevorzugt ist ein 2-Komponenten-Kit, dessen 1. Komponente aus einem die Verbindungen der Formel (1), die Verbindungen der Formel (IIa) oder (IIb) und das Oxidationsmittel sowie gegebenenfalls weitere übliche pulverförmige kosmetische Zusatzstoffe enthaltenden Pulver besteht, und dessen 2. Komponente Wasser oder eine flüssige kosmetische Zubereitung ist.

Die Zubereitungsform für die Komponenten (A1) und (A2) sowie das gebrauchsfertige Färbemittel (A) kann beispielsweise eine Lösung, insbesondere eine wässrige oder wässrig-alkoholische Lösung, eine Creme, ein Gel oder eine Emulsion sein. Ihre Zusammensetzung stellt eine Mischung der Verbindung der Formel (I) beziehungsweise der Verbindung der Formel (IIa) oder (IIb), und gegebenfalls eines Oxidationsmittels, mit den für solche Zubereitungen üblichen Zusätzen dar.

Übliche in Färbemitteln verwendete Zusätze in Lösungen, Cremes, Emulsionen, Gelen oder Aerosolschäumen sind zum Beispiel Lösungsmittel wie Wasser, niedere aliphatische Alkohole, beispielsweise Ethanol, n-Propanol und Isopropanol oder Glykole wie Glycerin und 1,2-Propandiol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohle, oxethylierte Nonylphenole, Fettsäurealkanolamide, oxethylierte Fettsäureester, ferner Verdicker wie höhere Fettalkohole, Stärke oder Cellulosederivate, Parfüme, Haarvorbehandlungsmittel, Konditionierer, Haarquellmittel, Konservierungsstoffe, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent (jeweils bezogen auf die Komponente (A1) bzw. (A2)), die Verdicker in einer Menge von etwa 0,1 bis 25 Gewichtsprozent (jeweils bezogen auf die Komponente (A1) bzw. (A2)) und die Pflegestoffe in einer Konzentration von etwa 0,1 bis 5,0 Gewichtsprozent (jeweils bezogen auf die Komponente (A1) bzw. (A2)).

Der pH-Wert des gebrauchsfertigen Färbemittels (A) beträgt jeweils etwa 6 bis 12, vorzugsweise etwa 7 bis 11. Zur Einstellung des für die Färbung gewünschten pH-Wertes des gebrauchsfertigen Färbemittels (A) können alkalisierende Mittel, wie zum Beispiel Ammoniak, Aminosäuren, Alkanolamine, Alkalihydroxide, Erdalkalihydroxide, Alkaliacetate, Erdalkaliacetate, Ammoniumcarbonate, Alkalicarbonate, Erdalkalicarbonate, Alkalisilikate, Erdalkalisilikate oder Ammoniumsilikate, oder Säuren, wie zum Beispiel Milchsäure, Essigsäure, Weinsäure, Phosphorsäure, Salzsäure, Zitronensäure, Ascorbinsäure oder Borsäure, zugesetzt werden.

Das gebrauchsfertige Färbemittel wird unmittelbar vor der Anwendung durch Vermischen der Komponenten (A1) und (A2) bzw. (A1) und (A2) und (A3) -gegebenenfalls unter Zusatz eines Alkalisierungsmittel oder einer Säure- hergestellt und sodann auf die Faser, insbesondere menschliche Haare, aufgetragen. Je nach gewünschter Farbtiefe läßt man diese Mischung etwa 5 bis 60 Minuten, vorzugsweise etwa 15 bis 30 Minuten, bei einer Temperatur von etwa 20 bis 50 °C, insbesondere bei etwa 30 bis 40 °C einwirken. Anschließend wird die Faser mit Wasser gespült, gegebenenfalls mit einem Shampoo gewaschen und sodann getrocknet.

Das erfindungsgemäße Färbemittel ermöglicht eine gleichmäßige, intensive, brillante und dauerhafte Färbung der Fasern, insbesondere von Keratinfasern, wie zum Beispiel menschlichen Haaren.

Die nachfolgenden Beispiele sollen den Gegenstand näher erläutern, ohne ihn auf diese Beispiele zu beschränken.

### Beispiele

### Beispiel 1a: Synthese von 3,4-Dimethyl-2(3H)-thiazolon-hydrazon-Hydrochlorid

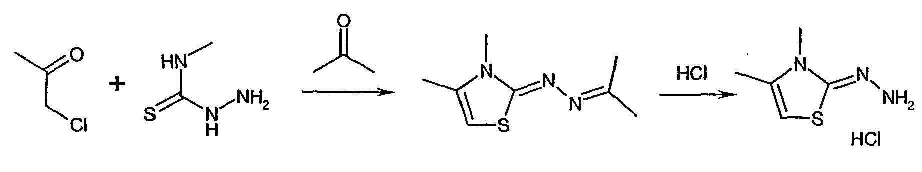

### Stufe A: 3,4-Dimethyl-2(3H)-thiazolon-(1-methylethyliden)hydrazon

21 g (200 mmol) 4-Methyl-3-thiosemicarbazid werden in 1000 ml Aceton 2 Stunden lang unter Rückfluss erwärmt. Dann wird die Lösung tropfenweise mit 20,4 g (220 mmol) Chloraceton versetzt. Die Reaktionsmischung wird sodann 7 Stunden lang unter Rückfluss erwärmt, und anschließend eingeengt. Das so erhaltene Rohprodukt wird aus Aceton umkristallisiert. Es werden 23 g eines orangefarbenen Pulvers (63% der Theorie) erhalten.
Schmelzpunkt: 139 - 139,6 °C
¹H-NMR (DMSO, 300 MHz): δ = 6,72 (s, breit, 1H, H-C(5)); δ = 3,67 (s, 3H, N-CH3); δ = 2,27 (d, J= 0,9 Hz, 3H, CH3-C(4)); δ = 2,17 (s, 3H, CH3); δ = 2,07 (s, 3H, CH3)
¹³C-NMR (DMSO, 300 MHz): δ = 169,16; 164,14; 139,02 (C(4)); 103,36 (C(5)); 34,47 (CH₃N); 24,60; 19,91; 13,53 (CH₃ (C4)).
MS (ESI): 184 (M⁺+1)

### Stufe B: 3,4-Dimethyl-2(3H)-thiazolon-hydrazon-Hydrochlorid

3,5 g (19 mmol) 3,4-Dimethyl-2(3H)-thiazolon-(1-methylethyliden)hydrazon aus Stufe 1 werden in 60 ml 6M Salzsäure bei 50 °C 30 Minuten lang erwärmt. Die Reaktionsmischung wird anschließend eingeengt und das Rohprodukt sodann aus Ethanol umkristallisiert. Es werden 2 g (60% der Theorie) eines rosafarbenen Pulvers erhalten.
Schmelzpunkt: 156,4 - 156,6 °C
¹H-NMR (DMSO, 300 MHz): δ = 6,58 (q, J= 0,9 Hz, 1H, H-C(5)); δ = 3,41 (s, 3H, N-CH3); δ = 2,18 (d, J= 0,9 Hz, 3H, CH3-C(4)).
MS (ESI): 144 (M⁺ +1).
¹³C-NMR (DMSO, 300 MHz): δ = 172,30 (C(2)); 138,79 (C(4)); 101,43 (C(5)); 32,92 (CH₃N); 13,40 (CH₃ (C4)).

| CHN-Analyse: (C₅H₉N₃S (0,96 HCl) (0,5 EtOH)): | | | | | |
|---|---|---|---|---|---|
| | % C | % H | % N | %S | %Cl |
| berechnet: | 35,81 | 6,49 | 20,88 | 15,93 | 16,90 |
| gefunden: | 35,20 | 6,30 | 21,00 | 15,40 | 16,80 |

### Beispiel 1b: Synthese von 3,4,5-Trimethyl-2(3H)-thiazolon-hydrazon-Hydrochlorid

3,4,5-Trimethyl-2(3H)-thiazolon-hydrazon-Hydrochlorid wird in Analogie zum Beispiel (1a), aus 4-Methyl-3-thiosemicarbazid und 3-Chlor-2-butanon, vorbereitet.
¹H-NMR (DMSO/D₂O, 300 MHz): δ = 3,55 (s, 3H, N-CH₃); δ = 2,16 (s, 3H, CH₃; δ = 2,12 (s, 3H, CH₃).
ESI-MS: 157 [M]⁺ (100)

### Beispiele 2 - 4: Färbemittel mit 3-Methyl-2(3H)-benzothiazolon-hydrazon-Hydrochlorid

### Komponente (A1)

| | |
|---|---|
| 4,00 g | Decylpolyglucose, 50 %ige wässrige Lösung |
| 0,20 g | Ethylendiaminotetraessigsäure-Dinatriumsalz-Hydrat |
| 5,00 g | Ethanol |
| 0,58 g | 3-Methyl-2(3H)-benzothiazolon-hydrazon-Hydrochlorid Hydrat |
| ad 100,00 g | Wasser, entmineralisiert |

### Komponente (A2)

| | |
|---|---|
| Y g | Aromatisches Enamin der Formel (IIa) oder (IIb) gemäß Tabelle 1 |
| 0,40 g | Kaliumpersulfat |

Bei Raumtemperatur (20-25 °C) oder unter leichtem Erwärmen (35-40 °C) werden die vorstehend genannten Bestandteile homogen miteinander vermischt. Der pH-Wert des gebrauchsfertigen Färbemittels (A) wird mit Natronlauge, Natriumcarbonat oder Ammoniak auf den in der Tabelle 1 angegebenen Wert eingestellt.

Das gebrauchsfertige Färbemittel wird auf gebleichtes Haar aufgetragen und mit einem Pinsel gleichmäßig verteilt. Nach einer Einwirkungszeit von 30 Minuten bei 40 °C wird das Haar mit lauwarmem Wasser gespült, mit einem handelsüblichen Shampoo gewaschen, mit lauwarmem Wasser gespült, und sodann getrocknet.

Die Einsatzmenge des aromatischen Enamins der Formel (IIa) oder (IIb) sowie die erhaltenen Färbungen sind in der nachfolgenden Tabelle 1 zusammengefaßt.

**Tabelle 1:**

| **Bsp. Nr.** | **Verwendetes Enamin** | **pH-Wert** | **Farbton** |
|---|---|---|---|
| | **(Menge in g)** | | |
| **2** | 1,2,3,3-Tetramethyl-3H-indolium-chlorid (0,52 g) | 8,6 | rubinrot |
| **3** | 3-Ethyl-1,2,3-trimethyl-3H-indolium-chlorid (0,56 g) | 9,2 | rubinrot |
| **4** | 1-Ethyl-2,3,3-trimethyl-5-methoxy-3H-indolium-tetrafluoroborat (0,76 g) | 8,8 | himbeerrot |

### Beispiele 5-8: Färbemittel mit 3,4-Dimethyl-2(3H)-thiazolon-hydrazon Hydrochlorid

### Komponente (A1)

| | |
|---|---|
| 4,00 g | Decylpolyglucose, 50 %ige wässrige Lösung |
| 0,20 g | Ethylendiaminotetraessigsäure-Dinatriumsalz-Hydrat |
| 5,00 g | Ethanol |
| 0,45 g | 3,4-Dimethyl-2(3H)-thiazolon-hydrazon Hydrochlorid |
| ad 100,00 g | Wasser, entmineralisiert |

### Komponente (A2)

| | |
|---|---|
| Y g | Aromatisches Enamin der Formel (IIa) oder (IIb) gemäß Tabelle 2 |
| 0,40 g | Kaliumpersulfat |

Bei Raumtemperatur (20-25 °C) oder unter leichtem Erwärmen (35-40 °C) werden die vorstehend genannten beiden Komponenten homogen miteinander vermischt. Der pH-Wert des gebrauchsfertigen Färbemittels (A) wird mit Natronlauge, Natriumcarbonat oder Ammoniak auf den in der Tabelle 2 angegebenen Wert eingestellt.

Das gebrauchsfertige Färbemittel wird auf auf gebleichtes Haar aufgetragen und mit einem Pinsel gleichmäßig verteilt. Nach einer Einwirkungszeit von 30 Minuten bei 40 °C wird das Haar mit lauwarmem Wasser gespült, mit einem handelsüblichen Shampoo gewaschen, mit lauwarmem Wasser gespült und sodann getrocknet.

Die Einsatzmenge des aromatischen Enamins der Formel (IIa) oder (IIb) sowie die erhaltenen Färbungen sind in der nachfolgenden Tabelle 2 zusammengefaßt.

**Tabelle 2:**

| **Bsp. Nr.** | **Verwendetes Enamin** | **pH-Wert** | **Farbton** |
|---|---|---|---|
| | **(Menge in g)** | | |
| **5** | 1,2,3,3-Tetramethyl-3H-indolium-hydrogensulfat (0,68 g) | 9,2 | himberrot |
| **6** | 3-Ethyl-1,2,3-trimethyl-3H-indolium-chlorid (0,56 g) | 9,2 | himberrot |
| **7** | 1-Ethyl-2,3,3-trimethyl-5-methoxy-3H-indolium-tetra-fluoroborat (0,76 g) | 8,8 | violett |
| **8** | 3-Ethyl-2-methyl-benzothiazolium p-toluolsulfonat (0,87 g) | 8,8 | orange |

### Beispiele 9-11: Färbemittel mit 3,4,5-Trimethyl-2(3H)-thiazolon-hydrazon Hydrochlorid

### Komponente (A1)

| | |
|---|---|
| 4,00 g | Decylpolyglucose, 50 %ige wässrige Lösung |
| 0,20 g | Ethylendiaminotetraessigsäure-Dinatriumsalz-Hydrat |
| 5,00 g | Ethanol |
| 0,48 g | 3,4,5-Trimethyl-2(3H)-thiazolon-hydrazon Hydrochlorid |
| ad 100,00 g | Wasser, entmineralisiert |

### Komponente (A2)

| | |
|---|---|
| Y g | Aromatisches Enamin der Formel (IIa) oder (IIb) gemäß Tabelle 3 |
| 0,40 g | Kaliumpersulfat |

Bei Raumtemperatur (20-25 °C) oder unter leichtem Erwärmen (35-40 °C) werden die vorstehend genannten beiden Komponenten homogen miteinander vermischt. Der pH-Wert des gebrauchsfertigen Färbemittels (A) wird mit Natronlauge, Natriumcarbonat oder Ammoniak auf den in der Tabelle 3 angegebenen Wert eingestellt.

Das gebrauchsfertige Färbemittel wird auf gebleichtes Büffelhaar aufgetragen und mit einem Pinsel gleichmäßig verteilt. Nach einer Einwirkungszeit von 30 Minuten bei 40 °C wird das Haar mit lauwarmem Wasser gespült, mit einem handelsüblichen Shampoo gewaschen, mit lauwarmem Wasser gespült und sodann getrocknet.

Die Einsatzmenge des aromatischen Enamins der Formel (IIa) oder (IIb) sowie die erhaltenen Färbungen sind in der nachfolgenden Tabelle 3 zusammengefaßt.

**Tabelle 3:**

| **Bsp. Nr.** | **Verwendetes Enamin** | **pH-Wert** | **Farbton** |
|---|---|---|---|
| | **(Menge in g)** | | |
| **9** | 1,2,3,3-Tetramethyl-3H-indolium-hydrogensulfat (0,68 g) | 9,2 | pink |
| **10** | 3-Ethyl-1,2,3-trimethyl-3H-indolium-chlorid (0,56 g) | 9,2 | pink |
| **11** | 1-Ethyl-2,3,3-trimethyl-5-methoxy-3H-indolium-tetrafluoroborat (0,76 g) | 8,8 | violett |

### Beispiele 12-14: Färbemittel mit 3,4-Dimethyl-2(3H)-thiazolon-hydrazon Hydrochlorid

### Komponente (A1)

| | |
|---|---|
| 4,00 g | Decylpolyglucose, 50 %ige wässrige Lösung |
| 0,20 g | Ethylendiaminotetraessigsäure-Dinatriumsalz-Hydrat |
| 5,00 g | Ethanol |
| ad 100,00 g | Wasser, entmineralisiert |

### Komponente (A2)

| | |
|---|---|
| 0,45 g | 3,4-Dimethyl-2(3H)-thiazolon-hydrazon Hydrochlorid |
| Y g | Aromatisches Enamin der Formel (IIa) oder (IIb) gemäß Tabelle 4 |

Bei Raumtemperatur (20-25 °C) oder unter leichtem Erwärmen (35-40 °C) wird die Komponente (A2) in 84 g der Komponente (A1) gelöst und mit 16 g einer 6prozentigen wässrigen Wasserstoffperoxidlösung homogen vermischt. Der pH-Wert des gebrauchsfertigen Färbemittels (A) wird mit Natronlauge, Natriumcarbonat oder Ammoniak auf den in der Tabelle 4 angegebenen Wert eingestellt.

Das gebrauchsfertige Haarfärbemittel wird auf auf gebleichtes Haar aufgetragen und mit einem Pinsel gleichmäßig verteilt. Nach einer Einwirkungszeit von 30 Minuten bei 40 °C wird das Haar mit lauwarmem Wasser gespült, mit einem handelsüblichen Shampoo gewaschen, mit lauwarmem Wasser gespült und sodann getrocknet.

Die Einsatzmenge des aromatischen Enamins der Formel (IIa) oder (IIb) sowie die erhaltenen Färbungen sind in der nachfolgenden Tabelle 4 zusammengefaßt.

**Tabelle 4:**

| **Bsp. Nr.** | **Verwendetes Enamin** | **pH-Wert** | **Farbton** |
|---|---|---|---|
| | **(Menge in g)** | | |
| **12** | 1,2,3,3-Tetramethyl-3H-indolium-hydrogensulfat (0,68 g) | 9,6 | pink |
| **13** | 3-Ethyl-1,2,3-trimethyl-3H-indolium-chlorid (0,56 g) | 9,1 | himberrot |
| **14** | 1-Ethyl-2,3,3-trimethyl-5-methoxy-3H-indolium-tetrafluoroborat (0,76 g) | 9,2 | violett |

Alle in der vorliegenden Anmeldung angegebenen Prozentzahlen stellen -soweit nicht anders angegeben- Gewichtsprozente dar.

## Patentansprüche

1. Gebrauchsfertges Mittel zur Färbung von Keratinfasern,
**dadurch gekennzeichnet, dass** es (a) mindestens ein Hydrazon-Derivat der Formel (I) oder dessen physiologisch verträgliches Salz, 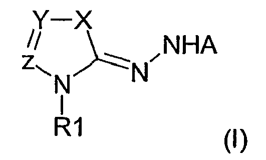 worin
**X** gleich Sauerstoff, Schwefel oder N-R2 ist,
**Y** gleich C-R3 oder Stickstoff ist und
**Z** gleich C-R4 oder Stickstoff ist,
mit der Bedingung, dass der hetererozyklische Teil der Verbindung der Formel (I) maximal drei Heteroatome enthält;
**A** Wasserstoff, eine Acetylgruppe, eine Trifluoracetylgruppe, eine Formylgruppe, eine (C₁-C₆)-Alkylsulfonylgruppe oder eine Arylsulfonylgruppe darstellt;
**R1** und **R2** gleich oder verschieden sein können, und unabhängig voneinander eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine Amino-(C₁-C₁₂)-alkylgruppe, eine Sulfonsäure-(C₁-C₁₂)-alkylgruppe, eine Formylgruppe, eine -C(O)-(C₁-C₁₂)-Alkylgruppe, eine -C(O)-Phenylgruppe, eine -C(O)NH-(C₁-C₁₂)-Alkylgruppe, eine -C(O)NH-Phenylgruppe, eine Phenylgruppe oder eine Benzylgruppe darstellen;
**R3** und **R4** gleich oder verschieden sein können und unabhängig voneinander Wasserstoff, ein Halogenatom, eine gesättigte oder ungesättigte (C₁-C₁₂)-Alkylgruppe, eine mit einem Halogenatom substituierte (C₁-C₁₂)-Alkylgruppe, eine Hydroxy-(C₁-C₁₂)-alkylgruppe, eine (C₁-C₁₂)-Alkoxygruppe, eine Cyanogruppe, eine Nitrogruppe, eine Aminogruppe, eine (C₁-C₁₂)-Alkylaminogruppe, eine Di(C₁-C₁₂)-alkylaminogruppe, eine Carboxylgruppe, eine -C(O)O-(C₁-C₁₂)-Alkylgruppe, eine -C(O)O-Phenylgruppe, eine Phenylgruppe oder eine Naphtylgruppe darstellen; und wenn Y und Z gleich C-R3 und C-R4 sind, **R3** und **R4** gemeinsam mit dem Restmolekül ein heterozyklisches oder carbozyklisches, gesättigtes oder ungesättigtes Ringsystem bilden können;
und (b) mindestens ein aromatisches Enamin der Formel (IIa) oder dessen Säureadditionssalze der Formel (IIb) 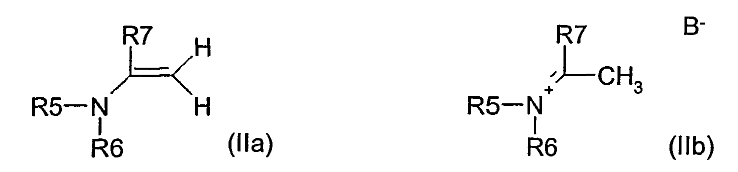
in der **R5** gleich einem ein- oder mehrkernigen aromatischen Rest ist;
**R6** gleich einer (C₁-C₁₂)-Alkylgruppe, einer Monohydroxy-(C₁-C₁₂)-alkylgruppe oder Mono-(C₁-C₆)-alkoxy-(C₁-C₆)-alkylgruppe, wobei zwischen den C-Atomen der Alkylkette Sauerstoffatome sitzen können, ist und
**R7** gleich einer (C₁-C₁₂)-Alkylgruppe, einer Mono-(C₁-C₆)-alkoxy-(C₁-C₆)-alkylgruppe, einer (C₁-C₆Alkylen-(C₁-C₆)-gruppe, einer (C₁-C₆)-Alkoxy-(C₁-C₆)-alkylengruppe, oder -O-, -NR8- oder -S- ist,
mit **R8** gleich einer (C₁-C₁₂)-Alkylgruppe, einer Mono-(C₁-C₆)-alkoxy-(C₁-C₆)-alkylgruppe, einer Monohydroxy-(C₁-C₁₂)-alkylgruppe oder Wasserstoff, wobei die Reste **R5** und **R7** gemeinsam mit dem Stickstoffatom und dem Kohlenstoffatom der Enamingrundstruktur eine zyklische Verbindung bilden können, und
**B⁻** gleich einem Anion einer organischen oder anorganischen Säure ist; und (c) mindestens ein Oxidationsmittel enthält.

2. Mittel nach Anspruch 1, **dadurch gekennzeichnet, dass** in Formel (1) gilt **X** gleich Schwefel, **Y** gleich C-R3, **Z** gleich C-R4 und **A** gleich Wasserstoff ist.

3. Mittel nach Anspruch 1 oder 2, **dadurch gekennzeichnet, dass** das Hydrazon-Derivat der Formel (I) ausgewählt ist aus 3-Methyl-2(3H)-thiazolon-hydrazon, 3,4-Dimethyl-2(3H)-thiazolon-hydrazon, 4-tert-Butyl-3-methyl-2(3H)-thiazolon-hydrazon, 3-Methyl-4-phenyl-2(3H)-thiazolon-hydrazon, 3-Methyl-4-(4-tolyl)-2(3H)-thiazolon-hydrazon, 4-(4-Methoxy)phenyl-3-methyl-2(3H)-thiazolon-hydrazon, 4-(4-Ethoxy)phenyl-3-methyl-2(3H)-thiazolon-hydrazon, 4-(4-Bromphenyl)-3-methyl-2(3H)-thiazolon-hydrazon, 4-(3-Bromphenyl)-3-methyl-2(3H)-thiazolon-hydrazon, 4-(4-Chlorphenyl)-3-methyl-2(3H)-thiazolon-hydrazon, 4-(3-Chlorphenyl)-3-methyl-2(3H)-thiazolon-hydrazon, 3-Methyl-4-(4-nitrophenyl)-2(3H)-thiazolon-hydrazon, 3-Methyl-4-(3-nitrophenyl)-2(3H)-thiazolon-hydrazon, 4-([1,1 '-Biphenyl]-4-yl)-3-methyl-2(3H)-thiazolon-hydrazon, 2-Hydrazono-2,3-dihydro-3-methyl-4-thiazolcarbonsäure-ethylester, 3,4,5-Trimethyl-2(3H)-thiazolon-hydrazon, 3,4-Dimethyl-5-phenyl-2(3H)-thiazolon-hydrazon, 3,5-Dimethyl-4-phenyl-2(3H)-thiazolon-hydrazon, 4,5-Diphenyl-3-methyl-2(3H)-thiazolon-hydrazon, 5-Ethyl-3-methyl-4-phenyl-2(3H)-thiazolon-hydrazon, 4-(4-Bromphenyl)-3-methyl-5-phenyl-2(3H)-thiazolon-hydrazon, 3-Methyl-5-phenyl-4-(4-tolyl)-2(3H)-thiazolon-hydrazon, 5-(4-Chlorphenyl)-4-phenyl-3-methyl-2(3H)-thiazolon-hydrazon, 5-(4-Chlorphenyl)-4-(4-methoxyphenyl)-3-methyl-2(3H)-thiazolon-hydrazon, 2-Hydrazono-2,3-dihydro-3,4-dimethyl-4-thiazolcarbonsäureethylester, 4-Amino-2-hydrazono-2,3-dihydro-3-methyl-5-thiazolcarbonitril, 4,5-Dimethyl-3-ethyl-2(3H)-thiazolon-hydrazon, 2-Hydrazono-2,3-dihydro-3-ethyl-4-methyl-thiazolcarbonsäureethylester, 5-Methyl-3-(1-methylethyl)-4-phenyl-2(3H)-thiazolon-hydrazon, 4,5-Diphenyl-3-(1-methylethyl)- 2(3H)-thiazolon-hydrazon, 4,5-Diphenyl-3-propyl-2(3H)-thiazolon-hydrazon, 3-Butyl-4,5-diphenyl-2(3H)-thiazolon-hydrazon, 4,5-Diphenyl-3-(2-methylpropyl)-2(3H)-thiazolon-hydrazon, 3-(2-Propenyl)-2(3H)-thiazolon-hydrazon, 4-Methyl-3-(2-propenyl)-2(3H)-thiazolon-hydrazon, 4-tert-Butyl-3-(2-propenyl)-2(3H)-thiazolon-hydrazon, 4-Phenyl-3-(2-propenyl)-2(3H)-thiazolon-hydrazon, 4,5-Diphenyl-3-(2-propenyl)-2(3H)-thiazolon-hydrazon, 3-Hydroxyethyl-2(3H)-thiazolon-hydrazon, 3-Hydroxyethyl-4-methyl-2(3H)-thiazolon-hydrazon, 3-Aminoethyl-2(3H)-thiazolon-hydrazon, 3-Aminoethyl-4-methyl-2(3H)-thiazolon-hydrazon, 3-Phenyl-2(3H)-thiazolon-hydrazon, 4-Methyl-3-phenyl-2(3H)-thiazolon-hydrazon, 3,4-Diphenyl-2(3H)-thiazolon-hydrazon, 4-p-Biphenylyl-3-phenyl-2(3H)-thiazolon-hydrazon, 4-(4-Methoxy)phenyl-3-phenyl-2(3H)-thiazolon-hydrazon, 4-tert-Butyl-3-phenyl-2(3H)-thiazolon-hydrazon, 3,4-Diphenyl-5-methyl-2(3H)-thiazolon-hydrazon, 3,4,5-Triphenyl-2(3H)-thiazolon-hydrazon, 4,5-Dimethyl-3-(phenylmethyl)-2(3H)-thiazolon-hydrazon, 2-Hydrazono-2,3-dihydro-3-[(phenylamino)-carbonyl]-4-methyl-thiazolcarbonsäureethylester, 3-Methyl-4,5,6,7-tetrahydro-2(3H)-benzothiazolon-hydrazon, 3-Methyl-2(3H)-benzothiazolon-hydrazon, 3,6-Dimethyl-2(3H)-benzothiazolon-hydrazon, 6-Chlor-3-methyl-2(3H)-benzothiazolon-hydrazon, 7-Chlor-3-methyl-2(3H)-benzothiazolon-hydrazon, 6-Hydroxy-3-methyl-2(3H)-benzothiazolon-hydrazon, 5-Methoxy-3-methyl-2(3H)-benzothiazolon-hydrazon, 7-Methoxy-3-methyl-2(3H)-benzothiazolon-hydrazon, 5,6-Dimethoxy-3-methyl-2(3H)-benzothiazolon-hydrazon, 5-Ethoxy-3-methyl-2(3H)-benzothiazolon-hydrazon, 6-Ethoxy-3-methyl-2(3H)-benzothiazolon-hydrazon, 3-Methyl-5-nitro-2(3H)-benzothiazolon-hydrazon, 3-Methyl-6-nitro-2(3H)-benzothiazolon-hydrazon, 5-Acetamido-3-methyl-2(3H)-benzothiazolon-hydrazon, 6-Acetamido-3-methyl-2(3H)-benzothiazolon-hydrazon, 5-Anilino-3-methyl-2(3H)-benzothiazolon-hydrazon, 6-Anilino-3-methyl-2(3H)-benzothiazolon-hydrazon, 2-Hydrazono-2,3-dihydro-3-methyl-6-benzothiazol-carbonsäure, 2-Hydrazono-2,3-dihydro-3-methyl-4-benzothiazol-sulfonsäure, 2-Hydrazono-2,3-dihydro-3-methyl-5-benzothiazol-sulfonsäure, 2-Hydrazono-2,3-dihydro-3-methyl-6-benzothiazol-sulfonsäure, 2-Hydrazono-2,3-dihydro-3-methyl-7-benzothiazol-sulfonsäure, 2-Hydrazono-2,3-dihydro-N,N,3-trimethyl-6-benzothiazol-sulfonsäureamid, [(2-Hydrazono-2,3-dihydro-3-methyl-6-benzothiazolyl)oxy]essigsäure-hydrazid, 3-Methyl-naphtho[2,3-d]thiazol-2(3H)-on-hydrazon, 3-Ethyl-2(3H)-benzothiazolon-hydrazon, 6-Ethoxy-3-ethyl-2(3H)-benzothiazolon-hydrazon, 3-Propyl-2(3H)-benzothiazolon-hydrazon, 3-Butyl-2(3H)-benzothiazolon-hydrazon, 3-Hexyl-2(3H)-benzothiazolon-hydrazon, 3-Hydroxyethyl-2(3H)-benzothiazolon-hydrazon, 3-Aminoethyl-2(3H)-benzothiazolon-hydrazon, 3-p-Methylbenzyl-2(3H)-benzothiazolon-hydrazon, 2-Hydrazono-2,3-dihydro-3-(2-hydroxyethyl)-6-benzothiazol-carbonsäure, 2-Hydrazono-2,3-dihydro-6-methoxy-3(2H)-benzothiazol-propansulfonsäure, 6-Hexadecyloxy-2-hydrazono-3(2H)-benzothiazol-propan-sulfonsäure, 2-Oxo-3-benzothiazolin-essigsäureethylester-hydrazon, 3-Acetyl-2(3H)-benzothiazolon-hydrazon und 2-Hydrazono-3(2H)-benzothiazol-carboxaldehyd.

4. Mittel nach Anspruch 1, **dadurch gekennzeichnet, dass** in Formel (lla)/(llb) **R5** und **R7** gemeinsam mit dem Stickstoffatom und dem Kohlenstoffatom der Enamingrundstruktur eine zyklische Verbindung bilden.

5. Mittel nach Anspruch 4, **dadurch gekennzeichnet, dass** R7 am aromatischen Rest **R5** mit dem in ortho-Stellung zum Enaminsubstituierten Kohlenstoff stehenden Kohlenstoffatom verbunden ist.

6. Mittel nach einem der Ansprüche 1, 4 oder 5, **dadurch gekennzeichnet, dass** das aromatische Enamin der Formel (IIa) oder (IIb) ausgewählt ist aus 1,2,3,3-Tetramethyl-3H-indolium-chlorid, 1,2,3,3-Tetramethyl-3H-indolium-bromid, 1,2,3,3-Tetramethyl-3H-indolium-hydrogensulfat, 1,2,3,3-Tetramethyl-3H-indolium-sulfat, 1,2,3,3-Tetramethyl-3H-indolium-tetrafluorborat, 3-Ethyl-1,2,3-trimethyl-3H-indolium-chlorid, 3-Ethyl-1,2,3-trimethyl-3H-indolium-bromid, 3-Ethyl-1,2,3-trimethyl-3H-indolium-sulfat, 3-Ethyl-1,2,3-trimethyl-3H-indolium-tetrafluorborat, 1-Ethyl-5-methoxy-2,3,3-trimethyl-3H-indolium-chlorid, 1-Ethyl-5-methoxy-2,3,3-trimethyl-3H-indolium-bromid, 1-Ethyl-5-methoxy-2,3,3-trimethyl-3H-indolium-sulfat, 1-Ethyl-5-methoxy-2,3,3-trimethyl-3H-indolium-tetrafluorborat, 5-Methoxy-1,2,3,3-tetramethyl-3H-indolium-chlorid, 5-Methoxy-1,2,3,3-tetramethyl-3H-indolium-bromid, 5-Methoxy-1,2,3,3-tetramethyl-3H-indolium-sulfat, 5-Methoxy-1,2,3,3-tetramethyl-3H-indolium-tetrafluorborat, 5-Nitro-1,2,3,3-tetramethyl-3H-indolium-chlorid, 5-Nitro-1,2,3,3-tetramethyl-3H-indolium-bromid, 5-Nitro-1,2,3,3-tetramethyl-3H-indolium-sulfat und 5-Nitro-1,2,3,3-tetramethyl-3H-indolium-tetrafluorborat, 2,3-Dimethylbenzothiazolium chorid, 2,3-Dimethylbenzothiazolium bromid, 2,3-Dimethylbenzothiazolium iodid, 2,3-Dimethylbenzothiazolium methylsulfat, 3-Ethyl-2-methyl-benzothiazolium chlorid, 3-Ethyl-2-methyl-benzothiazolium bromid, 3-Ethyl-2-methyl-benzothiazolium iodid, 3-Ethyl-2-methyl-benzothiazolium methylsulfat und 3-Ethyl-2-methyl-benzothiazolium p-toluolsulfonat.

7. Mittel nach Anspruch 1 bis 6, **dadurch gekennzeichnet, dass** das Oxidationsmittel ausgewählt ist aus Wasserstoffperoxid oder dessen Anlagerungsprodukte, Persalzen, Persäuren und enzymatischen Oxidationssystemen.

8. Mittel nach Anspruch 7, **dadurch gekennzeichnet, dass** das Oxidationsmittel ausgewählt ist aus Wasserstoffperoxid und dessen Anlagerungsprodukten und Persulfatsalzen.

9. Mittel nach einem der Ansprüche 1 bis 8, **dadurch gekennzeichnet, dass** es die Hydrazon-Derivate der Formel (I) und die aromatische Enamine der Formel (IIa) und (IIb) und das Oxidationsmittel jeweils in einer Gesamtmenge von 0,01 bis 10 Gewichtsprozent enthält.

10. Mittel nach einem der Ansprüche 1 bis 9, **dadurch gekennzeichnet, dass** es zusätzlich 0,01 bis 10 Gewichtsprozent eines physiologisch unbedenklichen, direktziehenden Farbstoffs aus der Gruppe der kationischen und anionischen Farbstoffe, der Dispersionsfarbstoffe, der Nitrofarbstoffe, der Azofarbstoffe, der Chinonfarbstoffe und der Triphenylmethanfarbstoffe enthält.

11. Mittel nach einem der Ansprüche 1 bis 10, **dadurch gekennzeichnet, dass** es einen pH-Wert von 7 bis 11 aufweist.

12. Mittel nach einem der Ansprüche 1 bis 11, **dadurch gekennzeichnet, dass** es ein Haarfärbemittel ist.

13. 2-Komponenten-Kit, bestehend aus einer Farbträgermasse (A1), welche die Verbindung der Formel (I) enthält, und einer weiteren Farbträgermasse (A2), welche das aromatische Enamin der Formel (IIa) oder (IIb) und ein Oxidationsmittel enthält.

14. 3-Komponenten-Kit, bestehend aus einer Farbträgermasse (A1), welche die Verbindung der Formel (I) enthält, einer weiteren Farbträgermasse (A2), welche das aromatische Enamin der Formel (IIa) oder (IIb) und ein Oxidationsmittel enthält, und einer 3. Komponente (A3), welche ein Mittel zur Einstellung des pH-Wertes enthält.

15. 2-Komponenten-Kit, bestehend aus einer pulverförmigen Farbträgermasse (A1), welche die Verbindungen der Formel (I), die Verbindungen der Formel (IIa) oder (IIb) und ein Oxidationsmittel sowie gegebenenfalls weitere übliche pulverförmige kosmetische Zusatzstoffe enthält, und einer flüssigen kosmetische Zubereitung (A2).

16. 3-Komponenten-Kit, bestehend aus einer Farbträgermasse (A1), welche die Verbindungen der Formel (I) enthält, einer weiteren Farbträgermasse (A2), welche die Verbindungen der Formel (IIa) oder (IIb) enthält, und einer ein Oxidationsmittel enthaltenden 3. Komponente (A3).

17. Verfahren zum Färben von Haaren bei dem ein Färbemittel nach einem der Ansprüche 1 bis 12 auf die Haare aufgetragen wird und nach einer Einwirkungszeit von 5 bis 60 Minuten bei einer Temperatur von 20 bis 50 °C das Haar mit Wasser gespült, gegebenenfalls mit einem Shampoo gewaschen und sodann getrocknet wird.

## Claims

1. Ready-to-use agent for dyeing keratin fibres, **characterized in that** it comprises (a) at least one hydrazone derivative of the formula (I) or physiologically compatible salt thereof, 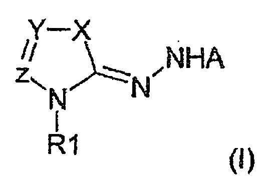 in which
**X** is oxygen, sulphur or N-R2,
**Y** is C-R3 or nitrogen and
**Z** is C-R4 or nitrogen,
with the condition that the heterocyclic moiety of the compound of the formula (I) contains at most three heteroatoms;
**A** is hydrogen, an acetyl group, a trifluoroacetyl group, a formyl group, a (C₁-C₆)-alkylsulphonyl group or an arylsulphonyl group;
**R1** and **R2** may be identical or different and, independently of one another, are a saturated or unsaturated (C₁-C₁₂)-alkyl group, a (C₁-C₁₂)-alkyl group substituted by a halogen atom, a hydroxy-(C₁-C₁₂)-alkyl group, an amino- (C₁-C₁₂) -alkyl group, a sulpho-(C₁-C₁₂)-alkyl group, a formyl group, a -C(O)-(C₁-C₁₂)-alkyl group, a -C(O)-phenyl group, a -C(O)NH-(C₁-C₁₂)-alkyl group, a -C(O)NH-phenyl group, a phenyl group or a benzyl group;
**R3** and **R4** may be identical or different and, independently of one another, are hydrogen, a halogen atom, a saturated or unsaturated (C₁-C₁₂)-alkyl group, a (C₁-C₁₂)-alkyl group substituted by a halogen atom, a hydroxy-(C₁-C₁₂)-alkyl group, a (C₁-C₁₂)-alkoxy group, a cyano group, a nitro group, an amino group, a (C₁-C₁₂)-alkylamino group, a di (C₁-C₁₂)-alkylamino group, a carboxyl group, a -C(O)O-(C₁-C₁₂)-alkyl group, a -C(O)O-phenyl group, a phenyl group or a naphthyl group; and if **Y** and **Z** are C-R3 and C-R4, **R3** and **R4,** together with the remainder of the molecule, can form a heterocyclic or carbocyclic, saturated or unsaturated ring system;
and (b) at least one aromatic enamine of the formula (IIa) or acid addition salts thereof of the formula (IIb) 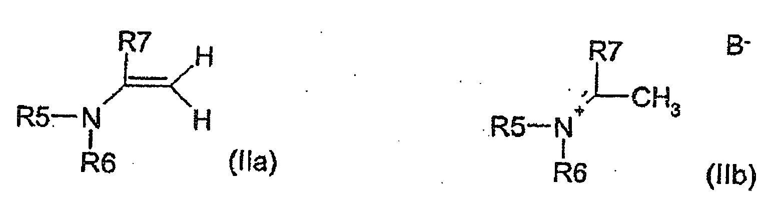
in which **R5** is a mono- or polynuclear aromatic radical;
**R6** is a (C₁-C₁₂)-alkyl group, a monohydroxy-(C₁-C₁₂)-alkyl group or mono-(C₁-C₆)-alkoxy-(C₁-C₆)-alkyl group, where oxygen atoms can sit between the C atoms of the alkyl chain, and
**R7** is a (C₁-C₁₂)-alkyl group, a mono-(C₁-C₆)-alkoxy-(C₁-C₆)-alkyl group, a (C₁-C₆)-alkylene-(C₁-C₆) group, a (C₁-C₆)-alkoxy-(C₁-C₆)-alkylene group, or -O-, -NR8- or -S-,
where **R8** is a (C₁-C₁₂)-alkyl group, a mono-(C₁-C₆)-alkoxy-(C₁-C₆)-alkyl group, a monohydroxy-(C₁-C₁₂)-alkyl group or hydrogen, where the radicals **R5** and **R7**, together with the nitrogen atom and the carbon atom of the enamine basic structure, can form a cyclic compound, and
**B⁻** is an anion of an organic or inorganic acid;
and (c) at least one oxidizing agent.

2. Agent according to Claim 1, **characterized in that**, in formula (I), **X** is sulphur, **Y** is C-R3, **Z** is C-R4 and **A** is hydrogen.

3. Agent according to Claim 1 or 2, **characterized in that** the hydrazone derivative of the formula (I) is selected from 3-methyl-2(3H)-thiazolone hydrazone, 3,4-dimethyl-2(3H)-thiazolone hydrazone, 4-tert-butyl-3-methyl-2(3H)-thiazolone hydrazone, 3-methyl-4-phenyl-2(3H)-thiazolone hydrazone, 3-methyl-4-(4-tolyl)-2(3H)-thiazolone hydrazone, 4-(4-methoxy)phenyl-3-methyl-2(3H)-thiazolone hydrazone, 4-(4-ethoxy)phenyl-3-methyl-2(3H)-thiazolone hydrazone, 4-(4-bromophenyl)-3-methyl-2(3H)-thiazolone hydrazone, 4-(3-bromophenyl)-3-methyl-2(3H)-thiazolone hydrazone, 4-(4-chlorophenyl)-3-methyl-2(3H)-thiazolone hydrazone, 4-(3-chlorophenyl)-3-methyl-2(3H)-thiazolone hydrazone, 3-methyl-4-(4-nitrophenyl)-2(3H)-thiazolone hydrazone, 3-methyl-4-(3-nitrophenyl)-2(3H)-thiazolone hydrazone, 4-([1,1'-biphenyl]-4-yl)-3-methyl-2(3H)-thiazolone hydrazone, 2-hydrazono-2,3-dihydro-3-methyl-4-thiazolecarboxylic ethyl ester, 3,4,5-trimethyl-2(3H)-thiazolone hydrazone, 3,4-dimethyl-5-phenyl-2(3H)-thiazolone hydrazone, 3,5-dimethyl-4-phenyl-2(3H)-thiazolone hydrazone, 4,5-diphenyl-3-methyl-2(3H)-thiazolone hydrazone, 5-ethyl-3-methyl-4-phenyl-2(3H)-thiazolone hydrazone, 4-(4-bromophenyl)-3-methyl-5-phenyl-2(3H)-thiazolone hydrazone, 3-methyl-5-phenyl-4-(4-tolyl)-2(3H)-thiazolone hydrazone, 5-(4-chlorophenyl)-4-phenyl-3-methyl-2(3H)-thiazolone hydrazone, 5-(4-chlorophenyl)-4-(4-methoxyphenyl)-3-methyl-2(3H)-thiazolone hydrazone, 2-hydrazono-2,3-dihydro-3,4-dimethyl-4-thiazolecarboxylic ethyl ester, 4-amino-2-hydrazono-2,3-dihydro-3-methyl-5-thiazolecarbonitrile, 4,5-dimethyl-3-ethyl-2(3H)-thiazolone hydrazone, 2-hydrazono-2,3-dihydro-3-ethyl-4-methylthiazolecarboxylic ethyl ester, 5-methyl-3-(1-methylethyl)-4-phenyl-2(3H)-thiazolone hydrazone, 4,5-diphenyl-3-(1-methylethyl)-2(3H)-thiazolone hydrazone, 4,5-diphenyl-3-propyl-2(3H)-thiazolone hydrazone, 3-butyl-4,5-diphenyl-2(3H)-thiazolone hydrazone, 4,5-diphenyl-3-(2-methylpropyl)-2(3H)-thiazolone hydrazone, 3-(2-propenyl)-2(3H)-thiazolone hydrazone, 4-methyl-3-(2-propenyl)-2(3H)-thiazolone hydrazone, 4-tert-butyl-3-(2-propenyl)-2(3H)-thiazolone hydrazone, 4-phenyl-3-(2-propenyl)-2(3H)-thiazolone hydrazone, 4,5-diphenyl-3-(2-propenyl)-2(3H)-thiazolone hydrazone, 3-hydroxyethyl-2(3H)-thiazolone hydrazone, 3-hydroxyethyl-4-methyl-2(3H)-thiazolone hydrazone, 3-aminoethyl-2(3H)-thiazolone hydrazone, 3-aminoethyl-4-methyl-2(3H)-thiazolone hydrazone, 3-phenyl-2(3H)-thiazolone hydrazone, 4-methyl-3-phenyl-2(3H)-thiazolone hydrazone, 3,4-diphenyl-2(3H)-thiazolone hydrazone, 4-p-biphenyl-3-phenyl-2(3H)-thiazolone hydrazone, 4-(4-methoxy)-phenyl-3-phenyl-2(3H)-thiazolone hydrazone, 4-tert-butyl-3-phenyl-2(3H)-thiazolone hydrazone, 3,4-diphenyl-5-methyl-2(3H)-thiazolone hydrazone, 3,4,5-triphenyl-2(3H)-thiazolone hydrazone, 4,5-dimethyl-3-(phenylmethyl)-2(3H)-thiazolone hydrazone, 2-hydrazono-2,3-dihydro-3-[(phenylamino)carbonyl]-4-methylthiazole-carboxylic ethyl ester, 3-methyl-4,5,6,7-tetrahydro-2(3H)-benzothiazolone hydrazone, 3-methyl-2(3H)-benzothiazolone hydrazone, 3, 6-dimethyl-2(3H)-benzo-thiazolone hydrazone, 6-chloro-3-methyl-2(3H)-benzothiazolone hydrazone, 7-chloro-3-methyl-2(3H)-benzothiazolone hydrazone, 6-hydroxy-3-methyl-2(3H)-benzothiazolone hydrazone, 5-methoxy-3-methyl-2(3H)-benzothiazolone hydrazone, 7-methoxy-3-methyl-2(3H)-benzothiazolone hydrazone, 5,6-dimethoxy-3-methyl-2(3H)-benzothiazolone hydrazone, 5-ethoxy-3-methyl-2(3H)-benzothiazolone hydrazone, 6-ethoxy-3-methyl-2(3H)-benzothiazolone hydrazone, 3-methyl-5-nitro-2(3H)-benzothiazolone hydrazone, 3-methyl-6-nitro-2(3H)-benzothiazolone hydrazone, 5-acetamido-3-methyl-2(3H)-benzothiazolone hydrazone, 6-acetamido-3-methyl-2(3H)-benzothiazolone hydrazone, 5-anilino-3-methyl-2(3H)-benzothiazolone hydrazone, 6-anilino-3-methyl-2(3H)-benzothiazolone hydrazone, 2-hydrazono-2,3-dihydro-3-methyl-6-benzothiazolecarboxylic acid, 2-hydrazono-2,3-dihydro-3-methyl-4-benzothiazolesulphonic acid, 2-hydrazono-2,3-dihydro-3-methyl-5-benzothiazole-sulphonic acid, 2-hydrazono-2,3-dihydro-3-methyl-6-benzothiazolesulphonic acid, 2-hydrazono-2,3-dihydro-3-methyl-7-benzothiazolesulphonic acid, 2-hydrazono-2,3-dihydro-N,N,3-trimethyl-6-benzothiazolesulphonamide, [(2-hydrazono-2,3-dihydro-3-methyl-6-benzoethiazolyl)-oxy]acetic hydrazide, 3-methylnaphtho[2,3-d]thiazole-2(3H)-one hydrazone, 3-ethyl-2(3H)-benzothiazolone hydrazone, 6-ethoxy-3-ethyl-2(3H)-benzothiazolone hydrazone, 3-propyl-2(3H)-benzothiazolone hydrazone, 3-butyl-2(3H)-benzothiazolone hydrazone, 3-hexyl-2(3H)-benzothiazolone hydrazone, 3-hydroxyethyl-2(3H)-benzothiazolone hydrazone, 3-aminoethyl-2(3H)-benzothiazolone hydrazone, 3-p-methylbenzyl-2(3H)-benzothiazolone hydrazone, 2-hydrazono-2,3-dihydro-3-(2-hydroxyethyl)-6-benzothiazolecarboxylic acid, 2-hydrazono-2,3-dihydro-6-methoxy-3(2H)-benzothiazole-propanesulphonic acid, 6-hexadecyloxy-2-hydrazono-3(2H)-benzothiazolepropanesulphonic acid, 2-oxo-3-benzothiazolineacetic ethyl ester hydrazone, 3-acetyl-2(3H)-benzothiazolone hydrazone and 2-hydrazono-3(2H)-benzothiazolecarboxaldehyde.

4. Agent according to Claim 1, **characterized in that**, in formula (IIa)/(IIb) **R5** and **R7**, together with the nitrogen atom and the carbon atom of the enamine basic structure, form a cyclic compound.

5. Agent according to Claim 4, **characterized in that R7** on the aromatic radical **R5** is bonded to the carbon atom in the ortho position relative to the enamine-substituted carbon.

6. Agent according to one of Claims 1, 4 or 5, **characterized in that** the aromatic enamine of the formula (IIa) or (IIb) is selected from 1,2,3,3-tetramethyl-3H-indolium chloride, 1,2,3,3-tetramethyl-3H-indolium bromide, 1,2,3,3-tetramethyl-3H-indolium hydrogensulphate, 1,2,3,3-tetramethyl-3H-indolium sulphate, 1,2,3,3-tetramethyl-3H-indolium tetrafluoroborate, 3-ethyl-1,2,3-trimethyl-3H-indolium chloride, 3-ethyl-1,2,3-trimethyl-3H-indolium bromide, 3-ethyl-1,2,3-trimethyl-3H-indolium sulphate, 3-ethyl-1,2,3-trimethyl-3H-indolium tetrafluoroborate, 1-ethyl-5-methoxy-2,3,3-trimethyl-3H-indolium chloride, 1-ethyl-5-methoxy-2,3,3-trimethyl-3H-indolium bromide, 1-ethyl-5-methoxy-2,3,3-trimethyl-3H-indolium sulphate, 1-ethyl-5-methoxy-2,3,3-trimethyl-3H-indolium tetrafluoroborate, 5-methoxy-1,2,3,3-tetramethyl-3H-indolium chloride, 5-methoxy-1,2,3,3-tetramethyl-3H-indolium bromide, 5-methoxy-1,2,3,3-tetramethyl-3H-indolium sulphate, 5-methoxy-1,2,3,3-tetramethyl-3H-indolium tetrafluoroborate, 5-nitro-1,2,3,3-tetramethyl-3H-indolium chloride, 5-nitro-1,2,3,3-tetramethyl-3H-indolium bromide, 5-nitro-1,2,3,3-tetramethyl-3H-indolium sulphate and 5-nitro-1,2,3,3-tetramethyl-3H-indolium tetrafluoroborate, 2,3-dimethylbenzothiazolium chloride, 2,3-dimethylbenzothiazolium bromide, 2,3-dimethylbenzothiazolium iodide, 2,3-dimethylbenzothiazolium methylsulphate, 3-ethyl-2-methylbenzothiazolium chloride, 3-ethyl-2-methylbenzothiazolium bromide, 3-ethyl-2-methylbenzothiazolium iodide, 3-ethyl-2-methylbenzothiazolium methylsulphate and 3-ethyl-2-methylbenzothiazolium p-toluenesulphonate.

7. Agent according to Claims 1 to 6, **characterized in that** the oxidizing agent is selected from hydrogen peroxide or its addition products, persalts, peracids and enzymatic oxidation systems.

8. Agent according to Claim 7, **characterized in that** the oxidizing agent is selected from hydrogen peroxide and its addition products and persulphate salts.

9. Agent according to one of Claims 1 to 8, **characterized in that** it comprises the hydrazone derivatives of the formula (I) and the aromatic enamines of the formula (IIa) and (IIb) and the oxidizing agent in each case in a total amount of from 0.01 to 10 per cent by weight.

10. Agent according to one of Claims 1 to 9, **characterized in that** it additionally comprises 0.01 to 10 per cent by weight of a physiologically acceptable, direct dye from the group of cationic and anionic dyes, disperse dyes, nitro dyes, azo dyes, quinone dyes and triphenylmethane dyes.

11. Agent according to one of Claims 1 to 10, **characterized in that** it has a pH of from 7 to 11.

12. Agent according to one of Claims 1 to 11, **characterized in that** it is a hair colorant.

13. 2-Component kit consisting of a colour carrier mass (A1) which comprises the compound of the formula (I), and a further colour carrier mass (A2) which comprises the aromatic enamine of the formula (IIa) or (IIb) and an oxidizing agent.

14. 3-Component kit consisting of a colour carrier mass (A1) which comprises the compound of the formula (I), a further colour carrier mass (A2) which comprises the aromatic enamine of the formula (IIa) or (IIb) and an oxidizing agent, and a 3^{rd} component (A3) which comprises an agent for adjusting the pH.

15. 2-Component kit consisting of a pulverulent colour carrier mass (A1) which comprises the compounds of the formula (I), the compounds of the formula (IIa) or (IIb) and an oxidizing agent, and also, if appropriate, further customary pulverulent cosmetic additives, and a liquid cosmetic preparation (A2).

16. 3-Component kit consisting of a colour carrier mass (A1) which comprises the compounds of the formula (I), a further colour carrier mass (II) which comprises the compounds of the formula (IIa) or (IIb), and a 3^{rd} component (A3) comprising an oxidizing agent.

17. Method for dyeing hair in which a colorant according to one of Claims 1 to 12 is applied to the hair and, after a contact time of from 5 to 60 minutes at a temperature of from 20 to 50°C, the hair is rinsed with water, if appropriate washed with a shampoo and then dried.

## Revendications

1. Composition prête à l'emploi pour la teinture de fibres de kératine, **caractérisée en ce qu'**elle contient (a) au moins un dérivé d'hydrazone de formule (I) ou un sel physiologiquement acceptable d'un tel dérivé, 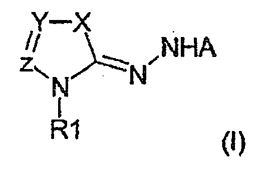 formule dans laquelle
**X** est un atome d'oxygène ou de soufre ou N-R2,
**Y** est C-R3 ou un atome d'azote et
**Z** est C-R4 ou un atome d'azote,
avec la condition que la partie hétérocyclique du composé de formule (I) contient au maximum trois hétéroatomes ;
**A** représente un atome d'hydrogène, un groupe acétyle, un groupe trifluoroacétyle, un groupe formyle, un groupe alkyl(C₁-C₆)sulfonyle ou un groupe arylsulfonyle ;
**R1** et **R2** peuvent être identiques ou différents et représentent, indépendamment l'un de l'autre, un groupe alkyle en C₁-C₁₂ saturé ou insaturé, un groupe alkyle en C₁-C₁₂ substitué par un atome d'halogène, un groupe hydroxyalkyle(C₁-C₁₂), un groupe aminoalkyle(C₁-C₁₂), un groupe sulfoalkyle(C₁-C₁₂), un groupe formyle, un groupe C(O)-alkyle (C₁-C₁₂), un groupe C(O)-phényle, un groupe C(O)NH-alkyle(C₁-C₁₂), un groupe C(O)NH-phényle, un groupe phényle ou un groupe benzyle ;
**R3** et **R4** peuvent être identiques ou différents et représentent, indépendamment l'un de l'autre, un atome d'hydrogène, un atome d'halogène, un groupe alkyle en C₁-C₁₂ saturé ou insaturé, un groupe alkyle en C₁-C₁₂ substitué par un atome d'halogène, un groupe hydroxyalkyle(C₁-C₁₂), un groupe alcoxy en C₁-C₁₂, un groupe cyano, un groupe nitro, un groupe amino, un groupe alkyl(C₁-C₁₂)amino, un groupe dialkyl(C₁-C₁₂)amino, un acide carboxy, un groupe C(O)O-alkyle(C₁-C₁₂) un groupe C(O)O-phényle, un groupe phényle ou un groupe naphtyle ; et lorsque **Y** et **Z** représentent C-R3 et C-R4, **R3** et
**R4** peuvent former ensemble avec la molécule restante un système cyclique hétérocyclique ou carbocyclique, saturé ou insaturé ;
et (b) au moins une énamine aromatique de formule (IIa) ou ses sels d'addition avec des acides, de formule (IIb) 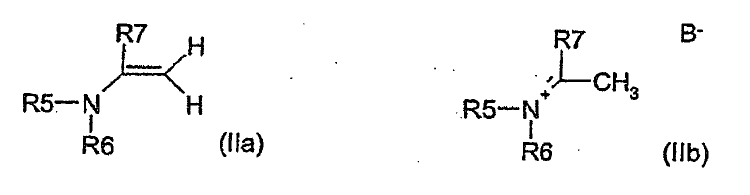
formules dans lesquelles **R5** est un radical aromatique mono- ou polynucléaire ;
**R6** représente un groupe alkyle en C₁-C₁₂, un groupe monohydroxyalkyle(C₁-C₁₂) ou un groupe monoalcoxy-(C₁-C₆) -alkyle (C₁-C₆) , des atomes d'oxygène pouvant être placés entre les atomes de carbone de la chaîne alkyle, et
**R7** représente un groupe alkyle en C₁-C₁₂, un groupe monoalcoxy(C₁-C₆) -alkyle (C₁-C₆), un groupe (C₁-C₆)-alkylène(C₁-C₆), un groupe alcoxy(C₁-C₆)-alkylène-(C₁-C₆), ou -O-, -NR8- ou -S-,
où **R⁸** représente un groupe alkyle en C₁-C₁₂, un groupe monoalcoxy(C₁-C₆)-alkyle(C₁-C₆), un groupe monohydroxyalkyle(C₁-C₁₂) ou un atome d'hydrogène, les radicaux **R5** et **R7** pouvant former ensemble avec l'atome d'azote et l'atome de carbone de la structure de base énamine un composé cyclique, et
**B⁻** représente un anion d'un acide organique ou minéral ;
et (c) au moins un oxydant.

2. Composition selon la revendication 1, **caractérisée en ce que** dans la formule (I) **X** représente un atome de soufre, **Y** représente C-R3, **Z** représente C-R4 et **A** représente un atome d'hydrogène.

3. Composition selon la revendication 1 ou 2, **caractérisée en ce que** le dérivé d'hydrazone de formule (I) est choisi parmi la 3-méthyl-2(3H)-thiazolone-hydrazone, la 3,4-diméthyl-2(3H)-thiazolone-hydrazone, la 4-tert-butyl-3-méthyl-2(3H)-thiazolone-hydrazone, la 3-méthyl-4-phényl-2(3H)-thiazolone-hydrazone, la 3-méthyl-4-(4-tolyl)-2(3H)-thiazolone-hydrazone, la 4-(4-méthoxy)phényl-3-méthyl-2(3H)-thiazolone-hydrazone, la 4-(4-éthoxy)phényl-3-méthyl-2(3H)-thiazolone-hydrazone, la 4-(4-bromophényl)-3-méthyl-2(3H)-thiazolone-hydrazone, la 4-(3-bromophényl)-3-méthyl-2(3H)-thiazolone-hydrazone, la 4-(4-chlorophényl)-3-méthyl-2(3H)-thiazolone-hydrazone, la 4-(3-chlorophényl)-3-méthyl-2(3H)-thiazolone-hydrazone, la 3-méthyl-4-(4-nitro-phényl)-2(3H)-thiazolone-hydrazone, la 3-méthyl-4-(3-nitrophényl)-2(3H)-thiazolone-hydrazone, la 4-([1,1'-biphényl]-4-yl)-3-méthyl-2(3H)-thiazolone-hydrazone, le 2-hydrazono-2,3-dihydro-3-méthyl-4-thiazolecarboxylate d'éthyle, la 3,4,5-triméthyl-2(3H)-thiazolone-hydrazone, la 3,4-diméthyl-5-phényl-2(3H)-thiazolone-hydrazone, la 3,5-diméthyl-4-phényl-2(3H)-thiazolone-hydrazone, la 4,5-diphényl-3-méthyl-2(3H)-thiazolone-hydrazone, la 5-éthyl-3-méthyl-4-phényl-2(3H)-thiazolone-hydrazone, la 4-(4-bromophényl)-3-méthyl-5-phényl-2(3H)-thiazolone-hydrazone, la 3-méthyl-5-phényl-4-(4-tolyl)-2(3H)-thiazolone-hydrazone, la 5-(4-chlorophényl)-4-phényl-3-méthyl-2(3H)-thiazolone-hydrazone, la 5-(4-chlorophényl)-4-(4-méthoxy-phényl)-3-méthyl-2(3H)-thiazolone-hydrazone, le 2-hydrazono-2, 3-dihydro-3, 4-diméthyl-4-thiazole-carboxylate d'éthyle, le 4-amino-2-hydrazono-2,3-dihydro-3-méthyl-5-thiazolecarbonitrile, la 4,5-diméthyl-3-éthyl-2(3H)-thiazolone-hydrazone, le 2-hydrazono-2, 3-dihydro-3-éthyl-4-méthyl-thiazole-carboxylate d'éthyle, la 5-méthyl-3-(1-méthyléthyl)-4-phényl-2(3H)-thiazolone-hydrazone, la 4,5-diphényl-3-(1-méthyléthyl)-2(3H)-thiazolone-hydrazone, la 4,5-diphényl-3-propyl-2(3H)-thiazolone-hydrazone, la 3-butyl-4,5-diphényl-2(3H)-thiazolone-hydrazone, la 4,5-diphényl-3-(2-méthylpropyl)-2(3H)-thiazolone-hydrazone, la 3-(2-propényl)-2(3H)-thiazolone-hydrazone, la 4-méthyl-3-(2-propényl-2(3H)-thiazolone-hydrazone, la 4-tert-butyl-3-(2-propényl)-2(3H)-thiazolone-hydrazone, la 4-phényl-3-(2-propényl)-2(3H)-thiazolone-hydrazone, la 4,5-diphényl-3-(2-propényl)-2(3H)-thiazolone-hydrazone, la 3-hydroxyéthyl-2(3H)-thiazolone-hydrazone, la 3-hydroxyéthyl-4-méthyl)-2(3H)-thiazolone-hydrazone, la 3-aminoéthyl)-2(3H)-thiazolone-hydrazone, la 3-aminoéthyl-4-méthyl)-2(3H)-thiazolone-hydrazone, la 3-phényl)-2(3H)-thiazolone-hydrazone, la 4-méthyl-3-phényl)-2(3H)-thiazolone-hydrazone, la 3,4-diphényl)-2(3H)-thiazolone-hydrazone, la 4-p-biphénylyl-3-phényl)-2(3H)-thiazolone-hydrazone, la 4-(4-méthoxy)phényl-3-phényl)-2(3H)-thiazolone-hydrazone, la 4-tert-butyl-3-phényl)-2(3H)-thiazolone-hydrazone, la 3,4-diphényl-5-méthyl)-2(3H)-thiazolone-hydrazone, la 3,4,5-triphényl)-2(3H)-thiazolone-hydrazone, la 4,5-diméthyl-3-(phénylméthyl)-2(3H)-thiazolone-hydrazone, le 2-hydrazono-2,3-dihydro-3-[(phénylamino)carbonyl]-4-méthylthiazolecarboxylate d'éthyle, la 3-méthyl-4,5,6,7-tétrahydro-2(3H)-benzothiazolone-hydrazone, la 3-méthyl-2(3H)-benzothiazolone-hydrazone, la 3,6-diméthyl-2(3H)-benzothiazolone-hydrazone, la 6-chloro-3-méthyl-2(3H)-benzothiazolone-hydrazone, la 7-chloro-3-méthyl-2(3H)-benzothiazolone-hydrazone, la 6-hydroxy-3-méthyl-2(3H)-benzothiazolone-hydrazone, la 5-méthoxy-3-méthyl-2(3H)-benzothiazolone-hydrazone, la 7-méthoxy-3-méthyl-2(3H)-benzothiazolone-hydrazone, la 5,6-diméthoxy-3-méthyl-2(3H}-benzothiazolone-hydrazone, la 5-éthoxy-3-méthyl-2(3H)-benzothiazolone-hydrazone, la 6-éthoxy-3-méthyl-2(3H)-benzothiazolone-hydrazone, la 3-méthyl-5-nitro-2(3H)-benzothiazolone-hydrazone, la 3-méthyl-6-nitro-2(3H)-benzothiazolone-hydrazone, la 5-acétamido-3-méthyl-2(3H}-benzothiazolone-hydrazone, la 6-acétamido-3-méthyl-2(3H)-benzothiazolone-hydrazone, la 5-anilino-3-méthyl-2(3H)-benzothiazolone-hydrazone, la 6-anilino-3-méthyl-2(3H)-benzothiazolone-hydrazone, l'acide 2-hydrazono-2,3-dihydro-3-méthyl-6-benzothiazole-carboxylique, l'acide 2-hydrazono-2,3-dihydro-3-méthyl-4-benzothiazole-sulfonique, l'acide 2-hydrazono-2, 3-dihydro-3-méthyl-5-benzothiazole-sulfonique, l'acide 2-hydrazono-2,3-dihydro-3-méthyl-6-benzothiazole-sulfonique, l'acide 2-hydrazono-2,3-dihydro-3-méthyl-7-benzothiazole-sulfonique, le 2-hydrazono-2,3-dihydro-N,N,3-triméthyl-6-benzothiazole-sulfonamide, le [(2-hydrazono-2,3-dihydro-3-méthyl-6-benzothiazolyl)-oxy]acéto-hydrazide, la 3-méthyl-naphto[2,3-d]-thiazol-2(3H)-one-hydrazone, la 3-éthyl-2(3H)-benzothiazolone-hydrazone, la 6-éthoxy-3-éthyl-2 (3H)-benzothiazolone-hydrazone, la 3-propyl-2 (3H)-benzothiazolone-hydrazone, la 3-butyl-2(3H)-benzothiazolone-hydrazone, la 3-hexyl-2(3H)-benzothiazolone-hydrazone, la 3-hydroxyéthyl-2 (3H)-benzothiazolone-hydrazone, la 3-aminoéthyl-2(3H)-benzothiazolone-hydrazone, la 3-p-méthyl-benzyl-2(3H)-benzothiazolone-hydrazone, l'acide 2-hydrazono-2, 3-dihydro-3- (2-hydroxyéthyl) -6-benzothiazole-carboxylique, l'acide 2-hydrazono-2,3-dihydro-6-méthoxy-3(2H)benzothiazole-propane-sulfonique, l'acide 6-hexadécyloxy-2-hydrazono-3(2H)-benzothiazole-propanesulfonique, la 2-oxo-3-benzothiazoline-acétate d'éthyle-hydrazone, la 3-acetyl-2(3H)-benzothiazolone-hydrazone et le 2-hydrazono-3(2H)-benzothiazole-carboxaldéhyde.

4. Composition selon la revendication 1, **caractérisée en ce que** dans la formule (IIa)/(IIb) **R5** et **R7** forment ensemble avec l'atome d'azote et l'atome de carbone de la structure de base énamine un composé cyclique.

5. Composition selon la revendication 4, **caractérisée en ce que R7** sur le radical aromatique **R5** est lié à l'atome de carbone qui se trouve en position ortho par rapport à l'atome de carbone substitué par l'énamine.

6. Composition selon l'une quelconque des revendications 1, 4 et 5, **caractérisée en ce que** l'énamine aromatique de formule (IIa) ou (IIb) est choisie parmi le chlorure de 1,2,3,3-tétraméthyl-3H-indolium, le bromure de 1,2,3,3-tétraméthyl-3H-indolium, l'hydrogénosulfate de 1,2,3,3-tétraméthyl-3H-indolium, le sulfate de 1,2,3,3-tétraméthyl-3H-indolium, le tétrafluoroborate de 1,2,3,3-tétraméthyl-3H-indolium, le chlorure de 3-éthyl-1,2,3-triméthyl-3H-indolium, le bromure de 3-éthyl-1,2,3-triméthyl-3H-indolium, le sulfate de 3-éthyl-1,2,3-triméthyl-3H-indolium, le tétrafluoroborate de 3-éthyl-1,2,3-triméthyl-3H-indolium, le chlorure de 1-éthyl-5-méthoxy-2,3,3-triméthyl-3H-indolium, le bromure de 1-éthyl-5-méthoxy-2,3,3-triméthyl-3H-indolium, le sulfate de 1-éthyl-5-méthoxy-2,3,3-triméthyl-3H-indolium, le tétrafluoroborate de 1-éthyl-5-méthoxy-2,3,3-triméthyl-3H-indolium, le chlorure de 5-méthoxy-1,2,3,3-tétraméthyl-3H-indolium, le bromure de 5-méthoxy-1,2,3,3-tétraméthyl-3H-indolium, le sulfate de 5-méthoxy-1,2,3,3-tétraméthyl-3H-indolium, le tétrafluoroborate de 5-méthoxy-1,2,3,3-tétraméthyl-3H-indolium, le chlorure de 5-nitro-1,2,3,3-tétraméthyl-3H-indolium, le bromure de 5-nitro-1,2,3,3-tétraméthyl-3H-indolium, le sulfate de 5-nitro-1,2,3,3-tétraméthyl-3H-indolium et le tétrafluoroborate de 5-nitroy-1,2,3,3-tétraméthyl-3H-indolium, le chlorure de 2,3-diméthylbenzothiazolium, le bromure de 2,3-diméthylbenzothiazolium, l'iodure de 2,3-diméthylbenzothiazolium, le méthylsulfate de 2,3-diméthylbenzothiazolium, le chlorure de 3-éthyl-2-méthylbenzothiazolium, le bromure de 3-éthyl-2-méthylbenzothiazolium, l'iodure de 3-éthyl-2-méthylbenzothiazolium, le méthylsulfate de 3-éthyl-2-méthylbenzothiazolium et le p-toluène-sulfonate de 3-éthyl-2-méthylbenzothiazolium.

7. Composition selon l'une quelconque des revendications 1 à 6, **caractérisée en ce que** l'oxydant est choisi parmi le peroxyde d'hydrogène ou ses produits d'addition, les persels, les peracides et les systèmes d'oxydation enzymatiques.

8. Composition selon la revendication 7, **caractérisée en ce que** l'oxydant est choisi parmi le peroxyde d'hydrogène et ses produits d'addition et les persulfates.

9. Composition selon l'une quelconque des revendications 1 à 8, **caractérisée en ce qu'**elle contient les dérivés d'hydrazone de formule (I) et les énamines aromatiques de formules (IIa) et (IIb) et l'oxydant chacun en une quantité totale de 0,01 à 10% en poids.

10. Composition selon l'une quelconque des revendications 1 à 9, **caractérisée en ce qu'**elle contient en outre de 0,01 à 10% en poids d'un colorant direct physioloiquement sans inconvénient, choisi dans le groupe des colorants cationiques et des colorants anioniques, des colorants en dispersion, des colorants nitrés, des colorants azoïques, des colorants quinoniques et des colorants triphénylméthane.

11. Composition selon l'une quelconque des revendications 1 à 10, **caractérisée en ce qu'**elle présente un pH de 7 à 11.

12. Composition selon l'une quelconque des revendications 1 à 11, **caractérisée en ce qu'**elle est une composition de teinture pour cheveux.

13. Nécessaire à 2 composants, constitué d'une matière colorante (A1) qui contient le composé de formule (I), et d'une autre matière colorante (A2) qui contient l'énamine aromatique de formule (IIa) ou (IIb) et un oxydant.

14. Nécessaire à 3 composants, constitué d'une matière colorante (A1) qui contient le composé de formule (I) d'une autre matière colorante (A2) qui contient l'énamine aromatique de formule (IIa) ou (IIb) et un oxydant, et d'un troisième composant (A3) qui contient un agent pour l'ajustement du pH.

15. Nécessaire à 2 composants, constitué d'une matière colorante pulvérulente (A1) qui contient les composés de formule (I) ; les composés de formule (IIa) ou (IIb) et un oxydant ainsi qu'éventuellement d'autres additifs cosmétiques pulvérulents usuels, et d'une préparation cosmétique liquide (A2).

16. Nécessaire à 3 composants, constitué d'une matière colorante (A1) qui contient les composés de formule (I), d'une autre matière colorante (A2) qui contient les composés de formule (IIa) ou (IIb), et d'un troisième composant (A3) contenant un oxydant.

17. Procédé pour la teinture des cheveux, dans lequel on applique sur les cheveux une composition de teinture selon l'une quelconque des revendications 1 à 12 et, après un temps d'action de 5 à 60 minutes à une température de 20 à 50 °C, on rince les cheveux à l'eau, éventuellement on les lave avec un shampooing et ensuite on les sèche.
